# EUROPEAN PATENT SPECIFICATION

(11) **EP 2 435 017 B1**
(45) Date of publication and mention of the grant of the patent: **23.10.2019**
(21) Application number: 10723020.3
(22) Date of filing: 19.05.2010
(51) Int. Cl.: A61K 8/37, A61Q 17/04, C07C 69/73

(54) **BENZYLIDENE MALONATES**
BENZYLIDIENMALONATES
MALONATES DE BENZYLIDÈNE

(30) Priority: 29.05.2009 EP 09161452
(43) Date of publication of application: 04.04.2012
(73) Proprietor: BASF SE, 67056 Ludwigshafen am Rhein (DE)
(72) Inventor: HANSCH, Markus, 79539 Lörrach (DE); EHLIS, Thomas, 79100 Freiburg (DE); WALLQUIST, Olof, CH-4103 Bottmingen (CH); WAGNER, Barbara, 79539 Lörrach (DE); HERZOG, Bernd, 79639 Grenzach-Wyhlen (DE); GIESINGER, Jochen, 79639 Grenzach-Wyhlen (DE)
(74) Representative: BASF IP Association
(86) International application number: PCT/EP2010/056843
(87) International publication number: WO 2010/136360

(56) References cited:
- WO-A1-2004/067539
- JP-A- 9 087 234
- US-A- 4 457 911
- US-A- 5 624 663

## Description

The present invention relates to the use of specific monomeric benzylidene malonates for cosmetic preparations and new monomeric benzylidene malonates.

It is well known that ultraviolet radiation (light) is harmful to human skin. Depending on the wavelength UV radiation causes different types of skin damage. UV-B radiation (about 290 to about 320 nm) is responsible for sunburn and can cause skin cancer. UV-A radiation (about 320 to about 400 nm) while producing tanning of the skin, contributes also to sunburn and the induction of skin cancers. Moreover, the harmful effects of the UV-B radiation may be aggravated by UV-A radiation.

Therefore, an effective sunscreen formulation preferably comprises both at least one UV-A and UV-B filter and a broad band UV filter covering the full range from about 290nm to about 400 nm to prevent the human skin from the damage of sunlight.

Besides their screening power on solar radiation UV filters must also have good resistance to water and perspiration and also satisfactory photostability.

Unfortunately, many effective organic UV filters have a poor oil-solubility at a certain concentration and tend to crystallization. As a consequence the UV protection efficacy is significantly decreased.

It is known that there are lipophilic UV filters like Butyl Methoxydibenzoylmethane (sold under the tradename "Parsol 1789" by DSM) which have the particularity and also the disadvantage of being solid at ambient temperature. As a result, their use in sunscreen cosmetic compositions implies certain constraints in terms of their formulation and their use, in particular the selection of specific suitable cosmetic solvents that afford a proper solubility of these UV filters. Thus, a UV filter should show high solubility in common cosmetic oils or should be a good solvent for other UV filters that show poor oil solubility.

Moreover the oil soluble UV filters should be included in cosmetic sun care products without any impact on the sensorial characteristic of the emulsion. For that reason the optimal distribution of the UV absorber within the hydro-lipid film left on the skin after spreading should be guaranteed.

US 4 457 911 A discloses topical sunscreen compositions characterised by contents of di-iso-butyl p-methoxy benzylidene malonate.

US 5 624 663 A discloses topical sunscreen compositions comprising dibenzoylmethane and di-alkyl p-methoxy benzylidene malonates, wherein the alkyl group is chosen from ethyl, isopropyl, 2-ethyl hexyl, and iso-amyl.

JP 9 087234 A discloses UV absorbers for skin or hair containing a specific benzalmalonate derivative.

It is therefore an object of the present invention to find UV absorber formulations which have improved properties regarding the UV absorber.

Surprisingly it has been found that specific monomeric benzylidene malonates have very good properties as cosmetic UV-B absorbers.

Therefore, the present invention relates to a composition for use in the protection of human skin against UV radiation comprising benzylidene malonates of formula (1) 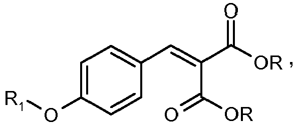 wherein
R₁ is methyl; and
R is

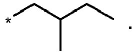

The compound according to the present invention corresponds to

| 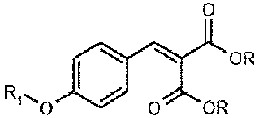 | | |
|---|---|---|
| | R₁ | R |
| MBM-09 | methyl | 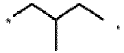 |

The benzylidene malonates according to the present invention are prepared in a manner known *per se* according to the following reaction scheme: 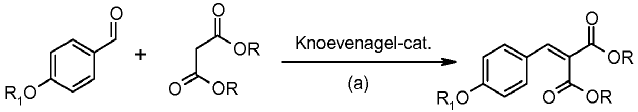 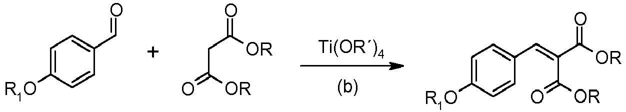 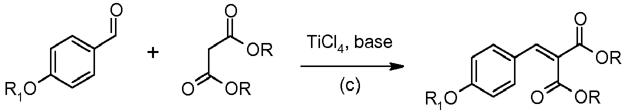 wherein R₁ is methyl; and R is

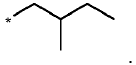

The Knoevenagel condensation under conditions (a) is carried out under azeotropic removal of water in the presence of a catalyst (literature: Jones, Gurnos. Knoevenagel condensation. Organic Reactions (New York) 15, 204-599 (1967).

Solvents used in this step are for example benzene, toluene, o-xylene, m-xylene, p-xylene, chloroform, dichloromethane, ethanol, methanol, tetrahydrofurane, acetonitrile, ethyl acetate, CCl₄, cyclohexane, n-hexane, n-pentane, or ionic liquids as for example 1-methyl-3-butyl imidazolium bromide. Mixtures of solvents can also be used.

The reaction temperature is preferably between 0°C and the reflux temperature of the solvent mixture, preferably between 0°C and 180°C, and more preferably between 20° and 150°C. The reaction time is preferably from 5min to 72h, and more preferably from 1 to10h.

The catalysts used in this reaction step are preferably primary, secondary or tertiary amines like piperidine, n-hexylamine, pyridine or triethylamine. The basic amines can be used as such or in combination with an acidic compound like acetic acid, benzoic acid or HCl. Suitable catalysts are any catalysts which are normally used in Knoevenagel reactions. Preferably are used catalysts such as a salt of an organic base with an organic acid, such as piperidinium acetate.

The Knoevenagel condensation under conditions (b) is carried out in the presence of a tetraalkyl orthotitanate Ti(OR') (0.5 req. to 5 eq. with respect to the aldehyde) as decribed for example in K. Yamashita et al., Tetrahedron 2005, 61, 7981-7985. Solvents used in this step are alcohols R'OH, for example ethanol, methanol, isopropanol, n-propanol, n-butanol, 2-methyl-1-butanol, isobutanol, 2-butanol, 2-pentanol. Other suitable solvents are benzene, toluene, o-xylene, m-xylene, p-xylene, chloroform, dichloromethane, tetrahydrofurane, acetonitrile, ethyl acetate, CCl₄, cyclohexane, n-hexane, n-pentane, or ionic liquids as for example 1-methyl-3-butyl imidazolium bromide. Mixtures of solvents can also be used.

The reaction temperature is preferably between -10°C and the reflux temperature of the solvent mixture, preferably between 0°C and 180°C, and more preferably between 20° and 150°C.

The Knoevenagel condensation under conditions (c) is carried out in the presence of a titanium tetrachloride TiCl₄ (0.5 req. to 5 eq. with respect to the aldehyde) as decribed for example in W. Lehnert, Tetrahedron Letters 1970, 54, 4723-4724 or in H. Chen et al., Eur. J. Org. Chem. 2006, 2329-2335. Solvents used in this step are ethers like tetrahydrofurane, dioxane, tert-butyl-methylether, diethyl ether, or alcohols R'OH, for example ethanol, methanol, isopropanol, n-propanol, n-butanol, 2-methyl-1-butanol, isobutanol, 2-butanol, 2-pentanol. Other suitable solvents are benzene, toluene, o-xylene, m-xylene, p-xylene, chloroform, dichloromethane, tetrahydrofurane, acetonitrile, ethyl acetate, CCl₄, cyclohexane, n-hexane, n-pentane, or ionic liquids as for example 1-methyl-3-butyl imidazolium bromide. Mixtures of solvents can also be used.

The reaction temperature is preferably between -10°C and the reflux temperature of the solvent mixture, preferably between 0°C and 180°C, and more preferably between 20° and 150°C.

The monomeric benzylidene malonates according to formula (1) are suitable especially as UV filters, that is to say for the protection of organic materials that are sensitive to ultraviolet light, especially human and animal skin and hair, against the action of UV radiation. Such compounds are accordingly suitable as light-protective agents in cosmetic, pharmaceutical and veterinary medicine preparations. Such compounds are preferably used in the dissolved state.

The invention accordingly relates also to a cosmetic preparation comprising the compound of formula (1), and cosmetically tolerable carriers or adjuvants.

The cosmetic preparation may also comprise, in addition to the UV absorber according to the invention, one or more further UV protective agents of the following substance classes: p-aminobenzoic acid derivatives, salicylic acid derivatives, benzophenone derivatives, dibenzoylmethane derivatives, diphenylacrylates, 3-imidazol-4-yl acrylic acid and esters; benzofuran derivatives, polymeric UV absorbers, cinnamic acid derivatives, camphor derivatives, hydroxyphenyltriazine compounds, benzotriazole compounds, trianilino-s-triazine derivatives, 2-phenylbenzimidazole-5-sulfonic acid and salts thereof; menthyl o-aminobenzoate; merocyanine derivatives; encapsulated UV absorbers, 4,4-diphenyl-1,3-butadiene derivatives, tris-(aryl) triazines, TiO₂, ZnO and mica.

The UV absorbers described in "Sunscreens", Eds. N.J. Lowe, N.A.Shaath, Marcel Dekker, Inc. , New York and Basle or in Cosmetics & Toiletries (107), 50ff (1992) also can be used as additional UV protective substances.

Special preference is given to the light-protective agents indicated in the following Table 2 (UV absorbers MBM-01-08 and 10-12 are included for illustrative purposes and are not according to the invention):

| Table 2: Suitable UV filter substances and adjuvants which can be additionally used with the UV absorbers of formula MBM-01 - MBM-12 according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts (Mexoryl SL) | 56039-58-8 |
| 12 | Methyl N,N,N-trimethyl-4-[(4,7,7-trimethyl-3-oxobicyclo[2,2,1]hept-2-ylidene)methyl]anilinium sulphate (Mexoryl SO) | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |

| No. | Chemical Name | CAS No. |
|---|---|---|
| 28 | Menthyl salicylate | 89-46-3 |
| 29 | 2-Ethylhexyl 2-cyano,3,3-diphenylacrylate; Octocrylene | 6197-30-4 |
| 30 | 2-ethylhexyl4-(dimethylamino)benzoate | 21245-02-3 |
| 31 | 2-ethylhexyl4-methoxycinnamate; Octyl Methoxy Cinnamate | 5466-77-3 |
| 32 | 2- ethylhexyl salicylate | 118-60-5 |
| 34 | 4-aminobenzoic acid | 150-13-0 |
| 35 | Benzoic acid, 4-amino-, ethyl ester, polymer with oxirane | 113010-52-9 |
| 38 | 2-phenyl-1 H-benzimidazole-5-sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2-ylidene)methyl]phenyl]methyl]-, homopolymer | 147897-12-9 |
| 40 | Triethanolamine salicylate | 2174-16-5 |
| 41 | 3, 3'-(1,4-phenylenedimethylene)bis[7,7-dimethyl-2-oxo-bicyclo[2.2.1]heptane-1 methanesulfonic acid] (Cibafast H) | 90457-82-2 |
| 42 | Titanium dioxide (primary particle size 10 - 50 nm) For example T805 or Eusolex T-AVO, Eusolex T-2000, Titaniumdioxid VT 817 | 13463-67-7 |
| 44 | Zinc oxide (primary particle size 20-100 nm) For example Zinc oxide NDM, Zinc oxide Z-Cote HP1, Nanox Zinc oxide | 1314-13-2 |
| 47 | 1H-Benzimidazole-4,6-disulfonic acid, 2,2'-(1,4-phenylene)bis-, disodium salt | 180898-37-7 |
| 48 | Benzoic acid, 4,4'-[[6-[[4-[[(1,1-dimethylethyl)amino]carbonyl]-phenyl]amino]1,3,5-triazine-2,4-diyl]diimino]bis-, bis(2-ethylhexyl)-ester; diethylhexyl butamido triazone (Uvasorb HEB) | 154702-15-5 |
| 49 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| 50 | Dimethicodiethylbenzalmalonate; Polysilicone 15 (Parsol SLX) | 207574-74-1 |
| 51 | Benzenesulfonic acid, 3-(2H-benzotriazol-2-yl)-4-hydroxy-5-(1-methylpropyl)-, monosodium salt (Tinogard HS) | 92484-48-5 |
| 53 | 1-Dodecanaminium, N-[3-[[4-(dimethylamino)benzoyl]amino]propyl]-N,N-dimethyl-, salt with 4-methylbenzenesulfonic acid (1:1) (Escalol HP610) | 156679-41-3 |
| 54 | 1-Propanaminium, N,N,N-trimethyl-3-[(1-oxo-3-phenyl-2-propenyl)-amino]-, chloride | 177190-98-6 |
| 55 | 1H-Benzimidazole-4,6-disulfonic acid, 2,2'-(1,4-phenylene)bis- | 170864-82-1 |
| 56 | 1,3,5-Triazine, 2,4,6-tris(4-methoxyphenyl)- | 7753-12-0 |
| 57 | 1,3,5-Triazine, 2,4,6-tris[4-[(2-ethylhexyl)oxy]phenyl]- | 208114-14-1 |
| 58 | 1-Propanaminium, 3-[[3-[3-(2H-benzotriazol-2-yl)-5-(1,1-dimethylethyl)-4-hydroxyphenyl]-1-oxopropyl]amino]-N, N-diethyl-N-methyl-, methyl sulfate (salt) | 340964-15-0 |
| 59 | 2-Propenoic acid, 3-(1 H-imidazol-4-yl)- | 104-98-3 |
| 60 | Benzoic acid, 2-hydroxy-, [4-(1-methylethyl)phenyl]methyl ester | 94134-93-7 |
| 61 | 1,2,3-Propanetriol, 1-(4-aminobenzoate)(Glyceryl PABA) | 136-44-7 |
| 62 | Benzeneacetic acid, 3,4-dimethoxy-a-oxo- | 4732-70-1 |
| 63 | 2-Propenoic acid, 2-cyano-3,3-diphenyl-, ethyl ester | 5232-99-5 |
| 64 | Anthralinic acid, p-menth-3-yl ester | 134-09-8 |
| 65 | 2,2'-bis(1,4-phenylene)-1H-benzimidazole-4,6-disulphonic acid mono sodium salt or Disodium phenyl dibenzimidazole tetrasulfonate (Neo Heliopan AP) | 349580-12-7 |
| 69 | sterols (cholesterol, lanosterol, phytosterols), as described in WO0341675 | |
| 70 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes) | |
| 71 | alpha-lipoic-acid as described in DE 10229995 | |
| 72 | synthetic organic polymers as described in EP 1 371 358, [0033]-[0041] | |
| 73 | phyllosilicates as described in EP 1371357 [0034]-[0037] | |
| 74 | silica compounds as described in EP1371356, [0033]-[0041] | |
| 75 | inorganic particles as described in DE10138496 [0043]-[0055] | |
| 76 | latex particles as described in DE10138496 [0027]-[0040] | |
| 77 | 1 H-Benzimidazole-4,6-disulfonic acid, 2,2'-(1,4-phenylene)bis-, disodium salt; Bisimidazylate (Neo Heliopan APC) | 180898-37-7 |
| 82 | Di-2-ethylhexyl-3,5-dimethoxy-4-hydroxy-benzalmalonate (Oxynex ST, EMD Chemicals, as described in US 20040247536) | |
| 83 | T-LiteTM MAX: Titanium Dioxide (and) Dimethoxydiphenylsilane (and) Triethoxycaprylylsilane Crosspolymer (and) Hydrated Silica (and) Aluminum Hydroxyde | |
| 84 | T-Lite SF: Titanium Dioxide (and) Aluminum Hydroxide (and) Dimethicone/Methicone Copolymer | |
| 85 | T-Lite SF-S: Titanium Dioxide (and) Hydrated Silica (and) Dimethicone/ Methicone Copolymer (and) Aluminum Hydroxide | |
| 86 | Z-COTE® MAX: Zinc Oxide (and) Diphenyl Capryl Methicone | |
| 87 | Z-COTE HP1: Zinc Oxide (and) Triethoxycaprylylsilane | |
| 88 | 1,3,5-Triazine-2,4,6-triamine, N2,N4-bis[4-[5-(1,1-dimethylpropyl)-2-benzoxazolyl]phenyl]-N6-(2-ethylhexyl)- (Uvasorb K2A) | 288254-16-0 |
| 89 | 1,1-(2,2'-Dimethylpropoxy)carbonyl-4,4-dihenyl-1,3-butadiene | 363602-15-7 |
| 90 | UV filter capsules containing an organic sunscreen as described in DE102007035567 or WO 2009012871 | |
| 91 | Phenol, 2,2'-[6-(4-methoxyphenyl)-1,3,5-triazine-2,4-diyl]bis[5-[(2-ethylhexyl)oxy]- (Tinosorb S) | 187393-00-6 |
| 92 | 1,3-Propanedione, 1-[4-(1,1-dimethylethyl)phenyl]-3-(4-methoxyphenyl)- (Parsol 1789) | 70356-09-1 |
| 93 | Benzoic acid, 4,4',4"-(1,3,5-triazine-2,4,6-triyltriimino)tris-, 1,1',1"-tris(2-ethylhexyl) ester (Uvinul T 150) | 88122-99-0 |
| 94 | Benzoic acid, 2-[4-(diethylamino)-2-hydroxybenzoyl]-, hexyl ester (Uvinul A Plus) | 302776-68-7 |
| 95 | Benzenesulfonic acid, 5-benzoyl-4-hydroxy-2-methoxy-, sodium salt | 6628-37-1 |
| 96 | Micronized 2,2'-Methylenebis[4-(1,1,3,3-tetramethylbutyl)-6-(2H-benzotriazol-2-yl)phenol] (Tinosorb M) | 103597-45-1 |
| 97 | Zinc Oxide | 131413-2 |
| 98 | Micronized 2,4,6-Tris(p-biphenylyl)-s-triazine (Tinosorb A2B) | 31274-51-8 |
| 99 | Micronized Methanone, 1,1'-(1,4-piperazinediyl)bis[1-[2-[4-(diethylamino)-2-hydroxybenzoyl]phenyl]- | 919803-06-8 |

| Table 3: Suitable UV filter substances which can be additionally used with the UV absorbers according to the present invention | |
|---|---|
| (Abbreviations T: Table, R: row, Comp: compound, Ex: compound(s) of Patent Example, p: page; the generic scope of the UV absorbers is described in the left-hand column; specific compounds are indicated in the right-hand column) | |
| DE 10013318 | T 1 pp 8-9, all Examples pp 10-13, T 2 pp 13-14, all Examples p 14, Ex A, B, C, D, E, F pp 19-20 |
| DE102004038485A1 | Formula 1 on p 2; Ex 1-4 on p 13; |
| DE102004039281A1 | Formulas I-II on p 1; Ex Ia-Iae on pp 7-12; Ex IIa-IIm on pp 14-15; Ex 1-25 on pp 42-56; |
| DE102005047647 A1 | Formulas I and II on p 3; Ex Ia-Ih on pp 5-7; Ex IIa-IIb on p 7; |
| DE 10206562 A1 | Ex 1-3 p 10, Ex 4-7 p 11, Ex 8-15 pp 12-14 |
| DE 10238144 A1 | Ex on p 3-5; |
| DE 10331804 | T 1 p 4, T 2 + 3 p 5 |
| DE 19704990 A1 | Ex 1-2 on pp 6-7; |
| EP 613 893 | Ex 1-5 + 15, T 1, pp 6-8 |
| EP 0 998 900 A1 | Ex on pp 4-11 |
| EP 1 000 950 | Comp. In Table 1, pp 18-21 |
| EP 1 005 855 | T 3, p 13 |
| EP 1 008 586 | Ex 1-3, pp 13-15 |
| EP 1 008 593 | Ex 1-8, pp 4-5 |
| EP 1 027 883 | Compound VII, p 3 |
| EP 1 027 883 | Comp I-VI, p 3 |
| EP 1 028 120 | Ex 1-5, pp 5-13 |
| EP 1 059 082 | Ex 1; T 1, pp 9-11 |
| EP 1 060 734 | T 1-3, pp 11-14 |
| EP 1 064 922 | Compounds 1-34, pp 6-14 |
| EP 1 077 246 A2 | Ex 1-16 on pp 5-11; |
| EP 1 081 140 | Ex 1-9, pp 11-16 |
| EP 1 103 549 | Compounds 1-76, pp 39-51 |
| EP 1 108 712 | 4,5-Dimorpholino-3-hydroxypyridazine |
| EP 1 123 934 | T 3, p 10 |
| EP 1 129 695 | Ex 1-7, pp 13-14 |
| EP 1142930 A1 | Formulas on p 2; |
| EP 1 167 359 | Ex 1, p 11 and Ex 2, p 12 |
| EP 1 232 148 B1 | Ex 4-17 on pp 3-5; |
| EP 1 258 481 | Ex 1, pp 7,8 |
| EP 1 310 492 A1 | Ex 1-16 on pp 22-30 |
| EP 1 371 654 A1 | Ex on pp 5-7 |
| EP 1 380 583 A2 | Ex 1, p 6; |
| EP 1 423 351 A2 | Ex 1-16 on pp 31-37; |
| EP 1 423 371 A1 | T 1 on pp 4-8, Ex on p 9, Ex 1-9 on pp 36-42; |
| EP 1 454 896 A1 | Ex 1-5 on pp 10-13, Examples on pp 4-5; |
| EP 1 471 059 A1 | Ex 1-5 on pp 4-5; |
| EP 1484051 A2 | Formula III-VII on pp18-19, Ex 7-14 on pp 7-9, Ex 18-23 on pp 11-12, Ex 24-40 on pp 14-17; |
| EP 1648849 A2 | Formula 1 on p 4; Ex 1-2 on pp 13-17; Ex C10 and O10 on pp15-16; |
| EP 1747773 A2 | Formulas I-VI on pp 2-4; Ex I-XXIII on pp 23-26; |
| EP 420 707 B1 | Ex 3, p 13 (CAS Reg. No 80142-49-0) |
| EP 503 338 | T 1, pp 9-10 |
| EP 517 103 | Ex 3,4,9,10 pp 6-7 |
| EP 517 104 | Ex 1, T 1, pp 4-5; Ex 8, T 2, pp 6-8 |
| EP 528 380 A1 | Comp. 1-9 pp 6-9 |
| EP 626 950 | all compounds |
| EP 669 323 | Ex 1-3, p 5 |
| EP 743 309 A1 | Ex 1-12 on pp 18-24; |
| EP 780 382 | Ex 1-11, pp 5-7 |
| EP 823 418 | Ex 1-4, pp 7-8 |
| EP 826 361 | T 1, pp 5-6 |
| EP 832 641 | Ex 5+6 p 7; T 2, p 8 |
| EP 832 642 | Ex 22, T 3, pp 10-15; T 4, p 16 |
| EP 848944 A2 | Formulas I and II on p 1; Ex on p 8; Examples on p 10; |
| EP 852 137 | T 2, pp 41-46 |
| EP 858 318 | T 1, p 6 |
| EP 863 145 | Ex 1-11, pp 12-18 |
| EP 878 469 A1 | T 1, pp 5-7; |
| EP 895 776 | Comp. In rows 48-58, p 3; R 25+33, p 5 |
| EP 911 020 | T 2, pp 11-12 |
| EP 916 335 | T 2-4, pp 19-41 |
| EP 924 246 | T 2, p 9 |
| EP 933 376 | Ex 1-15, pp 10-21 |
| EP 944 624 | Ex 1+2, pp 13-15 |
| EP 945 125 | T 3 a+b, pp 14-15 |
| EP 95 097 | Ex 1, p4 |
| EP 967 200 | Ex 2; T 3-5, pp 17-20 |
| EP 969 004 | Ex 5, T 1, pp 6-8 |
| FR 2842806 A1 | Ex I p 10, Ex II p 12 |
| FR 2861075 A1 | Ex 1-3 on pp 12-14; |
| FR 2862641 | Formula 3 on p4; Ex A-J on pp 7-9; |
| FR 2869907 A1 | Formula 1 on p 6; T 1 on p 7-8; Ex 4-39 on pp 12-35; |
| FR 2886143 | Formula 2 on p 4 and 2' on p 5; Ex a-i on pp 7-9; |
| FR 2888113 | Formula 1 on p 2; Ex a-i on pp 3-4; Ex j-n on pp 7-8; |
| FR 2889520 A1 | Formula 4 (Iriflophenone) on p 10; |
| KR 2004025954 | all kojyl benzoate derivatives |
| JP 06135985 A2 | Formula 1 on p 2; Ex 1-8 on pp 7-8; |
| JP 2000319629 | CAS Reg Nos. 80142-49-0, 137215-83-9, 307947-82-6 |
| JP 2003081910 A | Ex on p 1; |
| JP 2005289916 A | Formula I on p 1; Ex Ia-Id on pp 2-3; |
| JP 2005290240 A | Formulas I on p 2, Ex II on p 2; |
| JP 2006131603 | Ex 2 on p 2, Formula 1 on p 2; |
| JP2006-233,181 | Formula 1 on p 2, Ex on p 8; |
| JP 2006335855 | Formula 1-4 on p 2; Ex on p 8; |
| JP 2007131612 | Formula 2 and 3 on p 2, Formula 6-17 pp 9-11; |
| US 2003/0053966A1 | Ex on pp 3-6 |
| US 2004057912 A1 | Ex on p 7-9, Ex 1 on p 10; |
| US 2004057914 A1 | Ex on p 8-12, Ex 1 on p 12; |
| US 2004/0057911A1 | Formula I and II on p 1; formula III and IV on p3; Ex 1-3 on pp 5-6; |
| US 2004/0071640A1 | Ex 1-12 on pp 4-7; |
| US 2004/0091433A1 | Ex 1-6 on pp 14-16; |
| US 2004/0136931A1 | Ex 1-3 on p 7; |
| US 2004/0258636A1 | Ex 1-11 on pp 9-15; |
| US 2005/0019278A1 | Ex 1-9 on pp 6-8; |
| US 2005/0136012A1 | Formula 1 on p 2; |
| US 2005/0136014A1 | Formula a-c on p 2; Examples on p 3; |
| US 2005/0201957A1 | Formula 1 on p1; Ex A, B, C, D, E, F, G on pp 2-3; |
| US 2005/0249681A1 | all compounds on pp 2-3, Ex 1 on p 6; |
| US 2005186157A1 | Formula 1 on p 1; Ex 1-6 on pp 2-4; |
| US 2005260144A1 | Formula I on p1; Formula II on p 3; Ex 1-10 on pp 8-11; |
| US 2006018848A1 | Ex a-p on pp 3-4; |
| US 2006045859A1 | Formula 1 on p 1; Ex 1-10 on pp 2-4; |
| US2006228311 A1 | Formula 1 on p 2, Ex 1 on p 10; |
| US 2007025930A1 | Formula 1 on p1; Ex 1-2 on pp 5-6; |
| US 5 635 343 | all compounds on pp 5-10 |
| US 5 332 568 | Ex 1, p 5, T 1+2, pp 6-8 |
| US 5 338 539 | Ex 1-9, pp 3+4 |
| US 5 346 691 | Ex 40, p 7; T 5, p 8 |
| US 5 801 244 | Ex 1-5, pp 6-7 |
| US 6613340 | Ex I, II pp 9-11, Examples on rows 28-53 p 6 |
| US 6 800 274 B2 | Formulas I-VI and IX-XII on pp 14-18; |
| US 6 890 520 B2 | Ex 1-10 on pp 6-9; |
| US 6926887 B2 | Ex A on pp5/6; Formulas I - VIII on pp 27-29; |
| US 6936735 B2 | Formulas 1-2 on p 2; formula 3-4 on p 6; |
| US 6962692 B2 | Formulas VII and VIII on p 6; Formulas I, II, IV-VI, IX, X on pp 14-16; Formula III on p 19; |
| US 7163673 B2 | Formula III on p 14, Ex 1 on p 7; |
| US 7217820 B2 | Formula on p 2, Ex 1 on p 8; |
| US 7217821 B2 | Formula on p 2, Ex 1-5 on pp 4-5; |
| WO 0149686 | Ex 1-5, pp 16-21 |
| WO 0168047 | Tables on pp 85-96 |
| WO 0181297 | Ex 1-3, pp 9-11 |
| WO 0191695 | Formula I on p 4, T on p 8 |
| WO 0202501 A1 | Ex la-c, p 5 |
| WO 02069926 A1 | Ex on p 9, Ex on pp 17-23 |
| WO 02072583 | T on pp 68-70 |
| WO 02080876 | Ex 1 on pp 7-9 |
| WO 0238537 | All compounds p 3, compounds on rows 1-10 p 4 |
| WO 03004557 A1 | Ex A1-A29 on pp 36-57; |
| WO 03007906 | Ex I-XXIII, pp 42-48 |
| WO 03086340 A1 | Ex B1-B3 on pp 16-17; |
| WO 03086341 A2 | Formula 2-21, pp 4-6; |
| WO 03092643 A1 | T on pp 34-35, compounds listed on p 16 |
| WO 03097577 A1 | Ex on pp 6-8; Ex 1-3 on pp 15-18; |
| WO 03104183 A1 | Formula I-IV on p 1; Ex 1-5 on pp 27-28; |
| WO 04000256 A1 | Ex 1-10 on pp 18-24 |
| WO 04007592 | Ex 1-9 on pp 18-24; |
| WO 04020398 A1 | Ex 1-3 on pp 14-17 |
| WO 04020398 A1 | Formulas I-VI on pp 21-24, Formula IX on p 25; |
| WO 04075871 | Ex 1-3 on pp 17-18; Ex 7-9 on pp 21-22; |
| WO 05009938 A2 | Formula I on p 1; Ex 1-2 on pp 14-15; |
| WO 05053631 A1 | Ex 1-6 on pp 26-28; |
| WO 05065154 A2 | Formula a-c on pp 5-6; |
| WO 05080341 A1 | Formula 1 on p 3; Examples on pp 9-13; |
| WO 05092282 | Ex 1-9 on pp 34-43; |
| WO 05100319 A1 | Formula I on p 3, Ex 1-22 on p 72-74; |
| WO 05107692 A1 | Formula 1 on p 2; Ex 1-9 on pp 27-29; |
| WO 05118562 A1 | Formula I on p 4; Ex Ia-Ig on p 5; |
| WO 05121108 A1 | Formula I on p 3; Formula la on p 5; T 1 on p 7; Ex 3-22 on pp 11-23; |
| WO 06009451 | T 1 on pp 5-8; Formulas III and UV0 on p 9; |
| WO 06016806 | T 1 on pp 6-7; T 2 on p 10; T 3 on p 11; T 4 on p 15; |
| WO 06032741 | Formulas 1-3 on p 1; Ex a-k on pp 5-7; Ex 1-4 on pp 18-20; |
| WO 06048159 | Ex 1-6 on pp 28-34; |
| WO 06064366 A1 | Formula I on p 3; Ex 1c on p 23; Ex 2-9 on pp 24-31; |
| WO 06099952 A2 | Formula on p 4; Ex 3-7 on pp 28-29; |
| WO 06114381 A1 | Formula 1 on p 2; Formula 103 on p 47; |
| WO 06128732 A1 | Formula 1 on p 6, Ex a-i on pp 10-12; |
| WO 06128920 A1 | Formula 1 on p 3; Formulas IA and IB on p 10, Ex 1-15 on pp 21-35; |
| WO 07017179 A1 | Formula I on p 5; Ex 1-5 on pp 52-57; |
| WO 07007283 A1 | Examples on p 13-14; |
| WO 9217461 | Ex 1-22, pp 10-20 |
| WO 9220690 | Polymeric Comp in Examples 3-6 |
| WO 9301164 | T 1+2, pp 13-22 |
| WO 9714680 | Ex 1-3, p 10 |
| US 7229609 B2 | Comp. A-G pp. 18-20 |
| US 7229610 B2 | Comp. A-J pp. 27-30 |
| DE 102005058542 A1 | Comp. Ia-If, pp7-8 |
| JP2007154111 | Compounds according to Formula I, II, III on page 6; Examples on pp 13-18 |
| JP2007112765 | Formula on page 4; Formula on page 8 |
| WO2007081209 | Ex 1-3 on pp 17-18 |
| WO2007060116 | Compounds according to Formula 2 on p 6 with n from 1 to 20 |
| US2007189990 | Ex 2 on p 3 |
| WO2007017179 | Ex 1-5 on pp 52-58 |
| WO2006111233 | Comp. Iah-Iag in the table on pp 10-16 |
| WO2007039110 | Comp. Ia-IIb on pp 7-9 |
| WO2007065524 | Comp. Ia-If on pp 8-9 |
| US2007185057 | Ex 1-10 on pp 10-12 |
| JP2007106701 | Formula II and III on p 2; Formulas on pp 8-11 |
| JP2007153860 | Comp. Ia-Ig and IIa-IIc on p 2. |
| WO2007077729 | Comp. according to Formula I on p 26 with R'= Et and R" = nBu |
| JP2007204378 | Comp. according to Formula on p 2 with R1 = R2 = OH and R3 = OMe and R4 = H |
| WO2006064366 | Ex 1-9 on pp 21-31 |
| US20070249853 | Ex 1-9 on pp 7-9 |
| US2007224147 | Comp a-k on pp 3-5 |
| JP2007262008 | Formula on p 2 |
| JP2007277209 | Comp Va-c, VIa-c, VIIa-g, VIIIa-g on p 3 |
| WO2006056297 | Ex A p 65 |
| WO2007121818 | Ex on p 54; Ex 2 on p 55; Formulas on pp 56-59 |
| WO2007121845 | Comp. 1-15 on pp 10-13 |
| WO2007144981 | Comp. on p 3 |
| JP2007314501 | Comp.Ia-Ie on p 2; Comp.IIa-IIe on p 2; Cmp. III-VI on p 4; Cmp. VII p 8 |
| WO2007128723 | Comp. Ia-Id on pp 73-74 |
| JP2007261977 | CAS-Reg. Nr. 908576-94-3 CAS-Reg. Nr. 941278-56-4 |
| US2007275090 | Ex 1-5 pp 14-17; all compounds on p 9. |
| JP2008007444 | Comp. 2 on p 2; Comp. 6 on p 7; |
| US7311896 | Comp. STR-01-STR13 on p 6 |
| JP2008019229 | Comp. IIa-IIe and IIIa-IIIe on p 2 |
| WO2007137128 | All formulas on pp 10-11 |
| US20080050319 | All formulars p 2 R 43; Ex. 1-9 on pp 9-11 |
| US7326407 | Ex. 1-5 on pp. 10-12. |
| JP2008007443 | Ex. 6 on p 9; Ex. 7 p 10; Ex. 8-25 on pp 10-17 |
| US200803813 | Comp. Ia-Iz on pp 3-8; comp. Iaa-Iae p 8; comp. Ila-IIM on pp 8-9 |
| EP1894936 | Ex 1 on p 13; Comp. 14 p 17; |
| WO2007006807 | Ex 1-49 on pp 60-90 |
| US7354571 | Comp. a-q on pp 4-7; Ex 1-13 on pp 15-22 |
| WO2008041525 | Comp. (3), (4), (5), (6) on p. 6 |
| DE102006037724 | Ex. 1 on p. 32 |
| US2007140996 | Ex. 1 on pp.5-6 |
| DE102006004327 | Ex. 1-7 on pp. 17-21 |
| US2008138303 | Comp. (a)-(m) on pp. 44-6 |
| WO2008067928 | Formula on p. 78 |
| EP1258481B1 | Ex. 1 on p. 7 |
| EP1900743 | Comp. 1 on p. 14 |
| EP1688453 | Ex-1-23on pp. 10-15 |
| ES2300199 | Comp. 1 on p. 13 |
| WO2008122329 | Ex. 1-2 on pp. 28-29 |
| WO2008123504 | Comp. (S-01)-(S-31) on pp. 17-21 |
| WO2008131921 | Ex. 1-8 on pp. 81-88 |
| WO2009012871 | Ex. 1-3 on pp. 57-60 |
| US2008299055 | Ex. 1-22 on pp. 3-8 |
| US2008253978 | Ex. 1-15 on pp. 7-14 |
| WO2009038463 | Comp. 4 on p. 33; Comp. 5 on p. 35; Ex. 2-9 on pp. 36-37 |

In addition, BEMT (Tinosorb S, Bis-Ethylhexyloxyphenol Methoxyphenyl Triazine) encapsulated in a polymer matrix, for example in PMMA, as described in IP.com Journal (2009), 9(1B), 17, can also be used as additional UV protective substance.

The following compounds can also be used as additional UV protective substances:

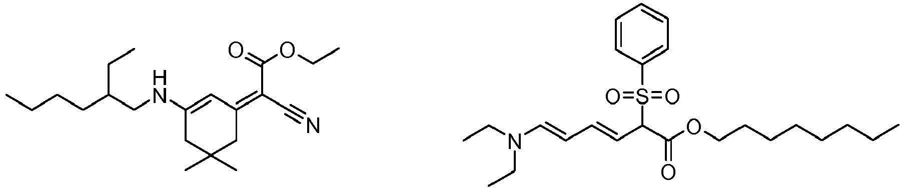

Each of the above-mentioned light-protective agents, especially the light-protective agents in the above Tables indicated as being preferred, can be used in admixture with the UV absorbers according to the invention. It will be understood in that connection that, in addition to the UV absorbers according to the invention, it is also possible for more than one of the additional light-protective agents to be used, for example, two, three, four, five or six further light-protective agents. Preference is given to the use of mixing ratios of UV absorbers according to the invention/further light-protective agents of from 1:99 to 99:1, especially from 1:95 to 95:1 and preferably from 10:90 to 90:10, based on weight. Of special interest are mixing ratios of from 20:80 to 80:20, especially from 40:60 to 60:40 and preferably of approximately 50:50. Such mixtures can be used, *inter alia,* to improve solubility or to increase UV absorption.

Appropriate mixtures can be used especially advantageously in a cosmetic composition according to the invention.

Preferably, the benzylidene malonates according to the present invention are used
- in combination with at least one additional oil soluble UV filter
- in combination with at least one additional water soluble UV filter
- in combination with at least one additional insoluble soluble UV filter
- in combination with at least one additional insoluble organic UV filter
- in combination with at least one additional insoluble inorganic UV filter
- in combination with at least one additional organic UV filter
- in combination with at least one additional inorganic UV filter
- in combination with at lest one additional organic/inorganid hybrid material UV filter
- in combination with at lest one additional encapsulated UV filter
- in combination with at lest one additional polymeric UV filter.

| Table 4: List of oil soluble organic UV filter combinations | | | | | | | | | | |
|---|---|---|---|---|---|---|---|---|---|---|
| Abbreviation | BP3 | BP4 | 3BC | BEMT | BMBM | DBT | DTS | EHT | MBC | PAMBC |
| Combination Nr. | | | | | | | | | | |
| UV SOL 1 | **x** | | | | | | | | | |
| UV SOL 2 | | **x** | | | | | | | | |
| UV SOL 3 | | | **x** | | | | | | | |
| UV SOL 4 | | | | **x** | | | | | | |
| UV SOL 5 | | | | | **x** | | | | | |
| UV SOL 6 | | | | | | **x** | | | | |
| UV SOL 7 | | | | | | | **x** | | | |
| UV SOL 8 | | | | | | | | **x** | | |
| UV SOL 9 | | | | | | | | | **x** | |
| UV SOL 10 | | | | | | | | | | **x** |
| UV SOL 11 | **x** | **x** | | | | | | | | |
| UV SOL 12 | **x** | | **x** | | | | | | | |
| UV SOL 13 | **x** | | | **x** | | | | | | |
| UV SOL 14 | **x** | | | | **x** | | | | | |
| UV SOL 15 | **x** | | | | | **x** | | | | |
| UV SOL 16 | **x** | | | | | | **x** | | | |
| UV SOL 17 | **x** | | | | | | | **x** | | |
| UV SOL 18 | **x** | | | | | | | | **x** | |
| UV SOL 19 | **x** | | | | | | | | | **x** |
| UV SOL 20 | | **x** | **x** | | | | | | | |
| UV SOL 21 | | **x** | | **x** | | | | | | |
| UV SOL 22 | | **x** | | | **x** | | | | | |
| UV SOL 23 | | **x** | | | | **x** | | | | |
| UV SOL 24 | | **x** | | | | | **x** | | | |
| UV SOL 25 | | **x** | | | | | | **x** | | |
| UV SOL 26 | | **x** | | | | | | | **x** | |
| UV SOL 27 | | **x** | | | | | | | | **x** |
| UV SOL 28 | | | **x** | **x** | | | | | | |
| UV SOL 29 | | | **x** | | **x** | | | | | |
| UV SOL 30 | | | **x** | | | **x** | | | | |
| UV SOL 31 | | | **x** | | | | **x** | | | |
| UV SOL 32 | | | **x** | | | | | **x** | | |
| UV SOL 33 | | | **x** | | | | | | **x** | |
| UV SOL 34 | | | **x** | | | | | | | **x** |
| UV SOL 35 | | | | **x** | **x** | | | | | |
| UV SOL 36 | | | | **x** | | **x** | | | | |
| UV SOL 37 | | | | **x** | | | **x** | | | |
| UV SOL 38 | | | | **x** | | | | **x** | | |
| UV SOL 39 | | | | **x** | | | | | **x** | |
| UV SOL 40 | | | | **x** | | | | | | **x** |
| UV SOL 41 | | | | | **x** | **x** | | | | |
| UV SOL 42 | | | | | **x** | | **x** | | | |
| UV SOL 43 | | | | | **x** | | | **x** | | |
| UV SOL 44 | | | | | **x** | | | | **x** | |
| UV SOL 45 | | | | | **x** | | | | | **x** |
| UV SOL 46 | | | | | | **x** | **x** | | | |
| UV SOL 47 | | | | | | **x** | | **x** | | |
| UV SOL 48 | | | | | | **x** | | | **x** | |
| UV SOL 49 | | | | | | **x** | | | | **x** |
| UV SOL 50 | | | | | | | **x** | **x** | | |
| UV SOL 51 | | | | | | | **x** | | **x** | |
| UV SOL 52 | | | | | | | **x** | | | **x** |
| UV SOL 53 | | | | | | | | **x** | **x** | |
| UV SOL 54 | | | | | | | | **x** | | **x** |
| UV SOL 55 | | | | | | | | | **x** | **x** |
| UV SOL 56 | **x** | **x** | **x** | | | | | | | |
| UV SOL 57 | **x** | **x** | | **x** | | | | | | |
| UV SOL 58 | **x** | **x** | | | **x** | | | | | |
| UV SOL 59 | **x** | **x** | | | | **x** | | | | |
| UV SOL 60 | **x** | **x** | | | | | **x** | | | |
| UV SOL 61 | **x** | **x** | | | | | | **x** | | |
| UV SOL 62 | **x** | **x** | | | | | | | **x** | |
| UV SOL 63 | **x** | **x** | | | | | | | | **x** |
| UV SOL 64 | **x** | | **x** | **x** | | | | | | |
| UV SOL 65 | **x** | | **x** | | **x** | | | | | |
| UV SOL 66 | **x** | | **x** | | | **x** | | | | |
| UV SOL 67 | **x** | | **x** | | | | **x** | | | |
| UV SOL 68 | **x** | | **x** | | | | | **x** | | |
| UV SOL 69 | **x** | | **x** | | | | | | **x** | |
| UV SOL 70 | **x** | | **x** | | | | | | | **x** |
| UV SOL 71 | **x** | | | **x** | **x** | | | | | |
| UV SOL 72 | **x** | | | **x** | | **x** | | | | |
| UV SOL 73 | **x** | | | **x** | | | **x** | | | |
| UV SOL 74 | **x** | | | **x** | | | | **x** | | |
| UV SOL 75 | **x** | | | **x** | | | | | **x** | |
| UV SOL 76 | **x** | | | **x** | | | | | | **x** |
| UV SOL 77 | **x** | | | | **x** | **x** | | | | |
| UV SOL 78 | **x** | | | | **x** | | **x** | | | |
| UV SOL 79 | **x** | | | | **x** | | | **x** | | |
| UV SOL 80 | **x** | | | | **x** | | | | **x** | |
| UV SOL 81 | **x** | | | | **x** | | | | | **x** |
| UV SOL 82 | **x** | | | | | **x** | **x** | | | |
| UV SOL 83 | **x** | | | | | **x** | | **x** | | |
| UV SOL 84 | **x** | | | | | **x** | | | **x** | |
| UV SOL 85 | **x** | | | | | | **x** | | | **x** |
| UV SOL 86 | **x** | | | | | | **x** | **x** | | |
| UV SOL 87 | **x** | | | | | | **x** | | **x** | |
| UV SOL 88 | **x** | | | | | | **x** | | | **x** |
| UV SOL 89 | **x** | | | | | | | **x** | **x** | |
| UV SOL 90 | **x** | | | | | | | **x** | | **x** |
| UV SOL 91 | **x** | | | | | | | | **x** | **x** |
| UV SOL 92 | | **x** | **x** | **x** | | | | | | |
| UV SOL 93 | | **x** | **x** | | **x** | | | | | |
| UV SOL 94 | | **x** | **x** | | | **x** | | | | |
| UV SOL 95 | | **x** | **x** | | | | **x** | | | |
| UV SOL 96 | | **x** | **x** | | | | | **x** | | |
| UV SOL 97 | | **x** | **x** | | | | | | **x** | |
| UV SOL 98 | | **x** | **x** | | | | | | | **x** |
| UV SOL 99 | | **x** | | **x** | **x** | | | | | |
| UV SOL 100 | | **x** | | **x** | | **x** | | | | |
| UV SOL 101 | | **x** | | **x** | | | **x** | | | |
| UV SOL 102 | | **x** | | **x** | | | | **x** | | |
| UV SOL 103 | | **x** | | **x** | | | | | **x** | |
| UV SOL 104 | | **x** | | **x** | | | | | | **x** |
| UV SOL 105 | | **x** | | | **x** | **x** | | | | |
| UV SOL 106 | | **x** | | | **x** | | **x** | | | |
| UV SOL 107 | | **x** | | | **x** | | | **x** | | |
| UV SOL 108 | | **x** | | | **x** | | | | **x** | |
| UV SOL 109 | | **x** | | | **x** | | | | | **x** |
| UV SOL 110 | | **x** | | | | **x** | **x** | | | |
| UV SOL 111 | | **x** | | | | **x** | | **x** | | |
| UV SOL 112 | | **x** | | | | **x** | | | **x** | |
| UV SOL 113 | | **x** | | | | **x** | | | | **x** |
| UV SOL 114 | | **x** | | | | | **x** | **x** | | |
| UV SOL 115 | | **x** | | | | | **x** | | **x** | |
| UV SOL 116 | | **x** | | | | | **x** | | | **x** |
| UV SOL 117 | | **x** | | | | | | **x** | **x** | |
| UV SOL 118 | | **x** | | | | | | **x** | | **x** |
| UV SOL 119 | | **x** | | | | | | | **x** | **x** |
| UV SOL 120 | | | **x** | **x** | **x** | | | | | |
| UV SOL 121 | | | **x** | **x** | | **x** | | | | |
| UV SOL 122 | | | **x** | **x** | | | **x** | | | |
| UV SOL 123 | | | **x** | **x** | | | | **x** | | |
| UV SOL 124 | | | **x** | **x** | | | | | **x** | |
| UV SOL 125 | | | **x** | **x** | | | | | | **x** |
| UV SOL 126 | | | **x** | | **x** | **x** | | | | |
| UV SOL 127 | | | **x** | | **x** | | **x** | | | |
| UV SOL 128 | | | **x** | | **x** | | | **x** | | |
| UV SOL 129 | | | **x** | | **x** | | | | **x** | |
| UV SOL 130 | | | **x** | | **x** | | | | | **x** |
| UV SOL 131 | | | **x** | | | **x** | **x** | | | |
| UV SOL 132 | | | **x** | | | **x** | | **x** | | |
| UV SOL 133 | | | **x** | | | **x** | | | **x** | |
| UV SOL 134 | | | **x** | | | **x** | | | | **x** |
| UV SOL 135 | | | **x** | | | | **x** | **x** | | |
| UV SOL 136 | | | **x** | | | | **x** | | **x** | |
| UV SOL 137 | | | **x** | | | | **x** | | | **x** |
| UV SOL 138 | | | **x** | | | | | **x** | **x** | |
| UV SOL 139 | | | **x** | | | | | **x** | | **x** |
| UV SOL 140 | | | **x** | | | | | | **x** | **x** |
| UV SOL 141 | | | | **x** | **x** | **x** | | | | |
| UV SOL 142 | | | | **x** | **x** | | **x** | | | |
| UV SOL 143 | | | | **x** | **x** | | | **x** | | |
| UV SOL 144 | | | | **x** | **x** | | | | **x** | |
| UV SOL 145 | | | | **x** | **x** | | | | | **x** |
| UV SOL 146 | | | | **x** | | **x** | **x** | | | |
| UV SOL 147 | | | | **x** | | **x** | | **x** | | |
| UV SOL 148 | | | | **x** | | **x** | | | **x** | |
| UV SOL 149 | | | | **x** | | **x** | | | | **x** |
| UV SOL 150 | | | | **x** | | | **x** | **x** | | |
| UV SOL 151 | | | | **x** | | | **x** | | **x** | |
| UV SOL 152 | | | | **x** | | | **x** | | | **x** |
| UV SOL 153 | | | | **x** | | | | **x** | **x** | |
| UV SOL 154 | | | | **x** | | | | **x** | | **x** |
| UV SOL 155 | | | | **x** | | | | | **x** | **x** |
| UV SOL 156 | | | | | **x** | **x** | **x** | | | |
| UV SOL 157 | | | | | **x** | **x** | | **x** | | |
| UV SOL 158 | | | | | **x** | **x** | | | **x** | |
| UV SOL 159 | | | | | **x** | **x** | | | | **x** |
| UV SOL 160 | | | | | **x** | | **x** | **x** | | |
| UV SOL 161 | | | | | **x** | | **x** | | **x** | |
| UV SOL 162 | | | | | **x** | | **x** | | | **x** |
| UV SOL 163 | | | | | **x** | | | **x** | **x** | |
| UV SOL 164 | | | | | **x** | | | **x** | | **x** |
| UV SOL 165 | | | | | **x** | | | | **x** | **x** |
| UV SOL 166 | | | | | | **x** | **x** | **x** | | |
| UV SOL 167 | | | | | | **x** | **x** | | **x** | |
| UV SOL 168 | | | | | | **x** | **x** | | | **x** |
| UV SOL 169 | | | | | | **x** | | **x** | **x** | |
| UV SOL 170 | | | | | | **x** | | **x** | | **x** |
| UV SOL 171 | | | | | | **x** | | | **x** | **x** |
| UV SOL 172 | | | | | | | **x** | **x** | **x** | |
| UV SOL 173 | | | | | | | **x** | **x** | | **x** |
| UV SOL 174 | | | | | | | **x** | | **x** | **x** |
| UV SOL 175 | | | | | | | | **x** | **x** | **x** |
| UV SOL 176 | **x** | **x** | **x** | **x** | | | | | | |
| UV SOL 177 | **x** | **x** | **x** | | **x** | | | | | |
| UV SOL 178 | **x** | **x** | **x** | | | **x** | | | | |
| UV SOL 179 | **x** | **x** | **x** | | | | **x** | | | |
| UV SOL 180 | **x** | **x** | **x** | | | | | **x** | | |
| UV SOL 181 | **x** | **x** | **x** | | | | | | **x** | |
| UV SOL 182 | **x** | | **x** | **x** | **x** | | | | | **x** |
| UV SOL 183 | **x** | | **x** | **x** | | **x** | | | | |
| UV SOL 184 | **x** | | **x** | **x** | | | **x** | | | |
| UV SOL 185 | **x** | | **x** | **x** | | | | **x** | | |
| UV SOL 186 | **x** | | **x** | **x** | | | | | **x** | |
| UV SOL 187 | **x** | | **x** | **x** | | | | | | **x** |
| UV SOL 188 | **x** | | | **x** | **x** | **x** | | | | |
| UV SOL 189 | **x** | | | **x** | **x** | | **x** | | | |
| UV SOL 190 | **x** | | | **x** | **x** | | | **x** | | |
| UV SOL 191 | **x** | | | **x** | **x** | | | | **x** | |
| UV SOL 192 | **x** | | | **x** | **x** | | | | | **x** |
| UV SOL 193 | **x** | | | | **x** | **x** | **x** | | | |
| UV SOL 194 | **x** | | | | **x** | **x** | | **x** | | |
| UV SOL 195 | **x** | | | | **x** | **x** | | | **x** | |
| UV SOL 196 | **x** | | | | **x** | **x** | | | | **x** |
| UV SOL 197 | **x** | | | | | **x** | **x** | **x** | | |
| UV SOL 198 | **x** | | | | | **x** | **x** | | **x** | |
| UV SOL 199 | **x** | | | | | **x** | **x** | | | **x** |
| UV SOL 200 | **x** | | | | | | **x** | **x** | **x** | |
| UV SOL 201 | **x** | | | | | | **x** | **x** | | **x** |
| UV SOL 202 | **x** | | | | | | | **x** | **x** | **x** |
| UV SOL 203 | | **x** | **x** | **x** | **x** | | | | | |
| UV SOL 204 | | **x** | **x** | **x** | | **x** | | | | |
| UV SOL 205 | | **x** | **x** | **x** | | | **x** | | | |
| UV SOL 206 | | **x** | **x** | **x** | | | | **x** | | |
| UV SOL 207 | | x | **x** | **x** | | | | | **x** | |
| UV SOL 208 | | **x** | **x** | **x** | | | | | | **x** |
| UV SOL 209 | | **x** | | **x** | **x** | | **x** | | | |
| UV SOL 210 | | **x** | | **x** | **x** | | | **x** | | |
| UV SOL 211 | | **x** | | **x** | **x** | | | | **x** | |
| UV SOL 212 | | **x** | | **x** | **x** | | | | | **x** |
| UV SOL 213 | | **x** | | | **x** | **x** | **x** | | | |
| UV SOL 214 | | **x** | | | **x** | **x** | | **x** | | |
| UV SOL 215 | | **x** | | | **x** | **x** | | | **x** | |
| UV SOL 216 | | **x** | | | **x** | **x** | | | | **x** |
| UV SOL 217 | | **x** | | | | **x** | **x** | **x** | | |
| UV SOL 218 | | **x** | | | | **x** | **x** | | **x** | |
| UV SOL 219 | | **x** | | | | **x** | **x** | | | **x** |
| UV SOL 220 | | **x** | | | | | **x** | **x** | **x** | |
| UV SOL 221 | | **x** | | | | | **x** | **x** | | **x** |
| UV SOL 222 | | **x** | | | | | | **x** | **x** | **x** |
| UV SOL 223 | | | **x** | **x** | **x** | **x** | | | | |
| UV SOL 224 | | | **x** | **x** | **x** | | **x** | | | |
| UV SOL 225 | | | **x** | **x** | **x** | | | **x** | | |
| UV SOL 226 | | | **x** | **x** | **x** | | | | **x** | |
| UV SOL 227 | | | **x** | **x** | **x** | | | | | **x** |
| UV SOL 228 | | | **x** | | **x** | **x** | | **x** | | |
| UV SOL 229 | | | **x** | | **x** | **x** | | | **x** | |
| UV SOL 230 | | | **x** | | **x** | **x** | | | | **x** |
| UV SOL 231 | | | **x** | | | **x** | **x** | **x** | | |
| UV SOL 232 | | | **x** | | | **x** | **x** | | **x** | |
| UV SOL 233 | | | **x** | | | **x** | **x** | | | **x** |
| UV SOL 234 | | | **x** | | | | **x** | **x** | **x** | |
| UV SOL 235 | | | **x** | | | | **x** | **x** | | **x** |
| UV SOL 236 | | | **x** | | | | | **x** | **x** | **x** |
| UV SOL 237 | | | | **x** | **x** | **x** | **x** | | | |
| UV SOL 238 | | | | **x** | **x** | **x** | | **x** | | |
| UV SOL 239 | | | | **x** | **x** | **x** | | | **x** | |
| UV SOL 240 | | | | **x** | **x** | **x** | | | | **x** |
| UV SOL 241 | | | | **x** | | **x** | **x** | | **x** | |
| UV SOL 242 | | | | **x** | | **x** | **x** | | | **x** |
| UV SOL 243 | | | | **x** | | | **x** | **x** | **x** | |
| UV SOL 244 | | | | **x** | | | **x** | **x** | | **x** |
| UV SOL 245 | | | | **x** | | | | **x** | **x** | **x** |
| UV SOL 246 | | | | | **x** | **x** | **x** | **x** | | |
| UV SOL 247 | | | | | **x** | **x** | **x** | | **x** | |
| UV SOL 248 | | | | | **x** | **x** | **x** | | | **x** |
| UV SOL 249 | | | | | **x** | | **x** | **x** | | **x** |
| UV SOL 250 | | | | | **x** | | | **x** | **x** | **x** |
| UV SOL 251 | | | | | | **x** | **x** | **x** | **x** | |
| UV SOL 252 | | | | | | **x** | **x** | **x** | | **x** |
| UV SOL 253 | | | | | | | **x** | **x** | **x** | **x** |

| Table 3 Abbreviations | | |
|---|---|---|
| BP3 | Benzophenone 3 | 131-57-7 |
| BP4 | Benzophenone-4 | 4065-45-6 |
| 3BC | 3-Benzydilene Camphor | 15087-24-8 |
| BEMT | Bis-Ethylhexyloxyphenol Methoxyphenyl Triazine | 103597-45-1 |
| BMBM | Butyl Methoxydibenzoylmethane | 70356-09-1 |
| DBT | Diethylhexyl Butamido Triazone | 154702-15-5 |
| DTS | Drometrizole Trisiloxane | 155633-54-8 |
| EHT | Ethylhexyl Triazone | 88122-99-0 |
| MBC | 4-Methylbenzylidene Camphor | 36861-47-9 |
| PAMBC | Polyacrylamido Methylbenzylidene Camphor | 147897-12-9 |

The following mixtures of oil miscible organic UV filters (Table 4) can be mixed together with the benzylidene malonates according to the present invention:

| Table 4: List of oil miscible organic UV filter combinations | | | | | | | | |
|---|---|---|---|---|---|---|---|---|
| Abbreviation | DHHB | EHDP | EHMC | EHS | HMS | IMC | OCR | PS15 |
| Combination Nr. | | | | | | | | |
| UV LIQ 1 | **x** | | | | | | | |
| UV LIQ 2 | | **x** | | | | | | |
| UV LIQ 3 | | | **x** | | | | | |
| UV LIQ 4 | | | | **x** | | | | |
| UV LIQ 5 | | | | | **x** | | | |
| UV LIQ 6 | | | | | | **x** | | |
| UV LIQ 7 | | | | | | | **x** | |
| UV LIQ 8 | | | | | | | | **x** |
| UV LIQ 9 | **x** | **x** | | | | | | |
| UV LIQ 10 | **x** | | **x** | | | | | |
| UV LIQ 11 | **x** | | | **x** | | | | |
| UV LIQ 12 | **x** | | | | **x** | | | |
| UV LIQ 13 | **x** | | | | | **x** | | |
| UV LIQ 14 | **x** | | | | | | **x** | |
| UV LIQ 15 | **x** | | | | | | | **x** |
| UV LIQ 16 | | **x** | **x** | | | | | |
| UV LIQ 17 | | **x** | | **x** | | | | |
| UV LIQ 18 | | **x** | | | **x** | | | |
| UV LIQ 19 | | **x** | | | | **x** | | |
| UV LIQ 20 | | **x** | | | | | **x** | |
| UV LIQ 21 | | **x** | | | | | | **x** |
| UV LIQ 22 | | | **x** | **x** | | | | |
| UV LIQ 23 | | | **x** | | **x** | | | |
| UV LIQ 24 | | | **x** | | | **x** | | |
| UV LIQ 25 | | | **x** | | | | **x** | |
| UV LIQ 26 | | | **x** | | | | | **x** |
| UV LIQ 27 | | | | **x** | **x** | | | |
| UV LIQ 28 | | | | **x** | | **x** | | |
| UV LIQ 29 | | | | **x** | | | **x** | |
| UV LIQ 30 | | | | **x** | | | | **x** |
| UV LIQ 31 | | | | | **x** | **x** | | |
| UV LIQ 32 | | | | | **x** | | **x** | |
| UV LIQ 33 | | | | | **x** | | | **x** |
| UV LIQ 34 | | | | | | **x** | **x** | |
| UV LIQ 35 | | | | | | **x** | | **x** |
| UV LIQ 36 | | | | | | | **x** | **x** |
| UV LIQ 37 | **x** | **x** | **x** | | | | | |
| UV LIQ 38 | **x** | **x** | | **x** | | | | |
| UV LIQ 39 | **x** | **x** | | | **x** | | | |
| UV LIQ 40 | **x** | **x** | | | | **x** | | |
| UV LIQ 41 | **x** | **x** | | | | | **x** | |
| UV LIQ 42 | **x** | **x** | | | | | | **x** |
| UV LIQ 43 | **x** | | **x** | **x** | | | | |
| UV LIQ 44 | **x** | | **x** | | **x** | | | |
| UV LIQ 45 | **x** | | **x** | | | **x** | | |
| UV LIQ 46 | **x** | | **x** | | | | **x** | |
| UV LIQ 47 | **x** | | **x** | | | | | **x** |
| UV LIQ 48 | **x** | | | **x** | **x** | | | |
| UV LIQ 49 | **x** | | | **x** | | **x** | | |
| UV LIQ 50 | **x** | | | **x** | | | **x** | |
| UV LIQ 51 | **x** | | | **x** | | | | **x** |
| UV LIQ 52 | **x** | | | | **x** | **x** | | |
| UV LIQ 53 | **x** | | | | **x** | | **x** | |
| UV LIQ 54 | **x** | | | | **x** | | | **x** |
| UV LIQ 55 | **x** | | | | | **x** | **x** | |
| UV LIQ 56 | **x** | | | | | **x** | | **x** |
| UV LIQ 57 | **x** | | | | | | **x** | **x** |
| UV LIQ 58 | | **x** | **x** | **x** | | | | |
| UV LIQ 59 | | **x** | **x** | | **x** | | | |
| UV LIQ 60 | | **x** | **x** | | | **x** | | |
| UV LIQ 61 | | **x** | **x** | | | | **x** | |
| UV LIQ 62 | | **x** | **x** | | | | | **x** |
| UV LIQ 63 | | **x** | | **x** | **x** | | | |
| UV LIQ 64 | | **x** | | **x** | | **x** | | |
| UV LIQ 65 | | **x** | | **x** | | | **x** | |
| UV LIQ 66 | | **x** | | **x** | | | | **x** |
| UV LIQ 67 | | **x** | | | **x** | **x** | | |
| UV LIQ 68 | | **x** | | | **x** | | **x** | |
| UV LIQ 69 | | **x** | | | **x** | | | **x** |
| UV LIQ 70 | | **x** | | | | **x** | **x** | |
| UV LIQ 71 | | **x** | | | | **x** | | **x** |
| UV LIQ 72 | | **x** | | | | | **x** | **x** |
| UV LIQ 73 | | | **x** | **x** | **x** | | | |
| UV LIQ 74 | | | **x** | **x** | | **x** | | |
| UV LIQ 75 | | | **x** | **x** | | | **x** | |
| UV LIQ 76 | | | **x** | **x** | | | | **x** |
| UV LIQ 77 | | | **x** | | **x** | **x** | | |
| UV LIQ 78 | | | **x** | | **x** | | **x** | |
| UV LIQ 79 | | | **x** | | **x** | | | **x** |
| UV LIQ 80 | | | **x** | | | **x** | **x** | |
| UV LIQ 81 | | | **x** | | | **x** | | **x** |
| UV LIQ 82 | | | **x** | | | | **x** | **x** |
| UV LIQ 83 | | | | **x** | **x** | **x** | | |
| UV LIQ 84 | | | | **x** | **x** | | **x** | |
| UV LIQ 85 | | | | **x** | **x** | | | **x** |
| UV LIQ 86 | | | | **x** | | **x** | **x** | |
| UV LIQ 87 | | | | **x** | | **x** | | **x** |
| UV LIQ 88 | | | | **x** | | | **x** | **x** |
| UV LIQ 89 | | | | | **x** | **x** | **x** | |
| UV LIQ 90 | | | | | **x** | **x** | | **x** |
| UV LIQ 91 | | | | | **x** | | **x** | **x** |
| UV LIQ 92 | | | | | | **x** | **x** | **x** |
| UV LIQ 93 | **x** | **x** | **x** | **x** | | | | |
| UV LIQ 94 | **x** | **x** | **x** | | **x** | | | |
| UV LIQ 95 | **x** | **x** | **x** | | | **x** | | |
| UV LIQ 96 | **x** | **x** | **x** | | | | **x** | |
| UV LIQ 97 | **x** | **x** | **x** | | | | | **x** |
| UV LIQ 98 | **x** | | **x** | **x** | | **x** | | |
| UV LIQ 99 | **x** | | **x** | **x** | | | **x** | |
| UV LIQ 100 | **x** | | **x** | **x** | | | | **x** |
| UV LIQ 101 | **x** | | | **x** | **x** | **x** | | |
| UV LIQ 102 | **x** | | | **x** | **x** | | **x** | |
| UV LIQ 103 | **x** | | | **x** | **x** | | | **x** |
| UV LIQ 104 | **x** | | | | **x** | **x** | **x** | |
| UV LIQ 105 | **x** | | | | **x** | **x** | | **x** |
| UV LIQ 106 | **x** | | | | | **x** | **x** | **x** |
| UV LIQ 107 | | **x** | **x** | **x** | **x** | | | |
| UV LIQ 108 | | **x** | **x** | **x** | | **x** | | |
| UV LIQ 109 | | **x** | **x** | **x** | | | **x** | |
| UV LIQ 110 | | **x** | **x** | **x** | | | | **x** |
| UV LIQ 111 | | **x** | | **x** | **x** | | **x** | |
| UV LIQ 112 | | **x** | | **x** | **x** | | | **x** |
| UV LIQ 113 | | **x** | | | **x** | **x** | **x** | |
| UV LIQ 114 | | **x** | | | **x** | **x** | | **x** |
| UV LIQ 115 | | **x** | | | | **x** | **x** | **x** |
| UV LIQ 116 | | | **x** | **x** | **x** | **x** | | |
| UV LIQ 117 | | | **x** | **x** | **x** | | **x** | |
| UV LIQ 118 | | | **x** | **x** | **x** | | | **x** |
| UV LIQ 119 | | | **x** | | **x** | **x** | | **x** |
| UV LIQ 120 | | | **x** | | | **x** | **x** | **x** |
| UV LIQ 121 | | | | **x** | **x** | **x** | **x** | |
| UV LIQ 122 | | | | **x** | **x** | **x** | | **x** |
| UV LIQ 123 | | | | | **x** | **x** | **x** | **x** |

| Table 4 abbreviations | | |
|---|---|---|
| Abbreviation | INCI name | Cas. No. |
| DHHB | Diethylamino Hydroxy Benzoyl Hexyl Benzoate | 302776-68-7 |
| EHDP | Ethylhexyl Dimethyl PABA | 21245-02-3 |
| EHMC | Ethylhexyl Methoxycinnamate | 5466-77-3 |
| EHS | Ethylhexyl Salicylate | 118-60-5 |
| HMS | Homosalate | 118-56-9 |
| IMC | Isoamyl p-Methoxycinnamate | 71617-10-2 |
| OCR | Octocrylene | 6197-30-4 |
| PS15 | Polysilicone-15 | 207574-74-1 |

The following mixtures of aqueous soluble or dispersible UV filters (Table 5) can be mixed together with the benzylidene malonates according to the present invention:

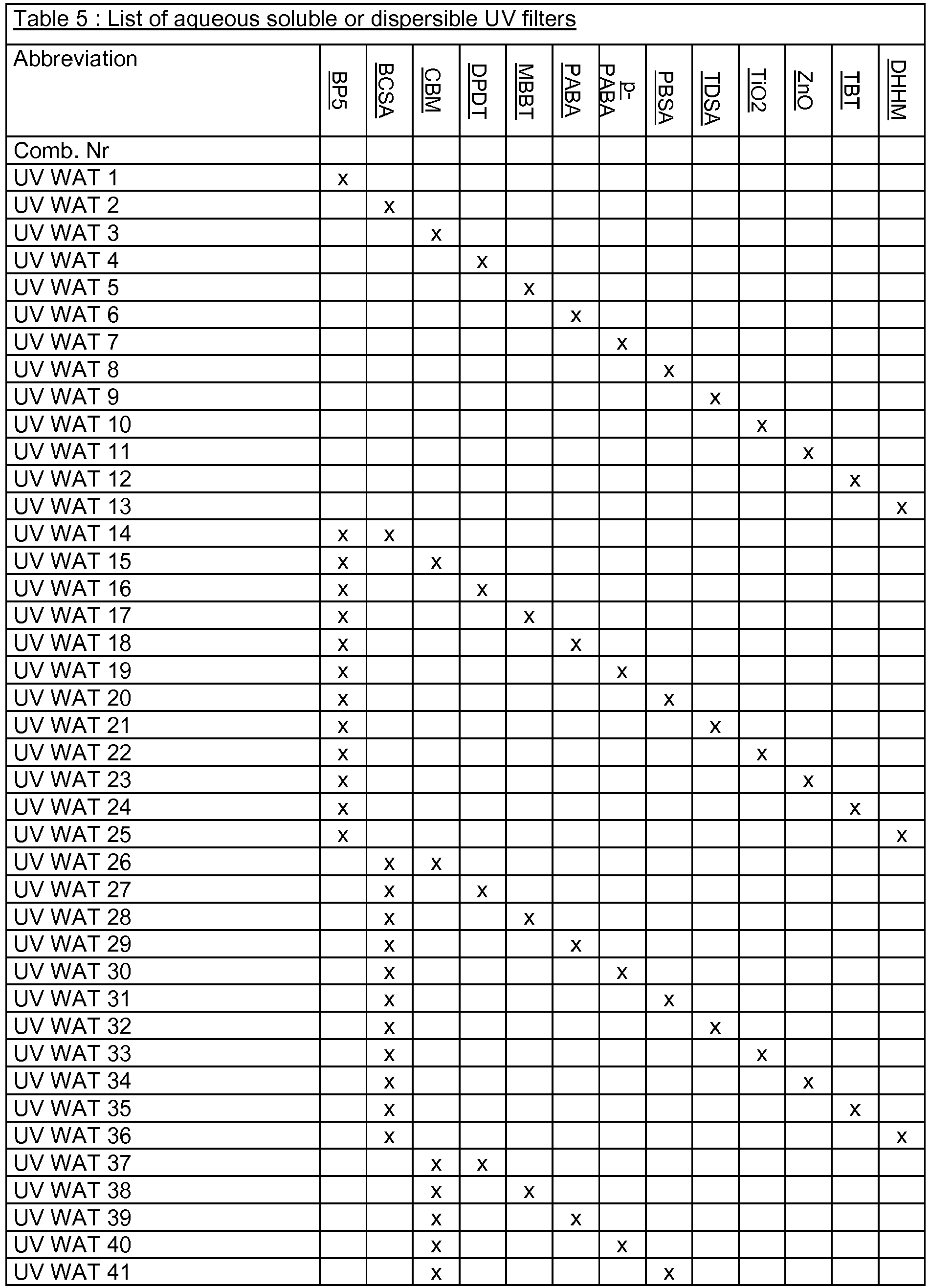

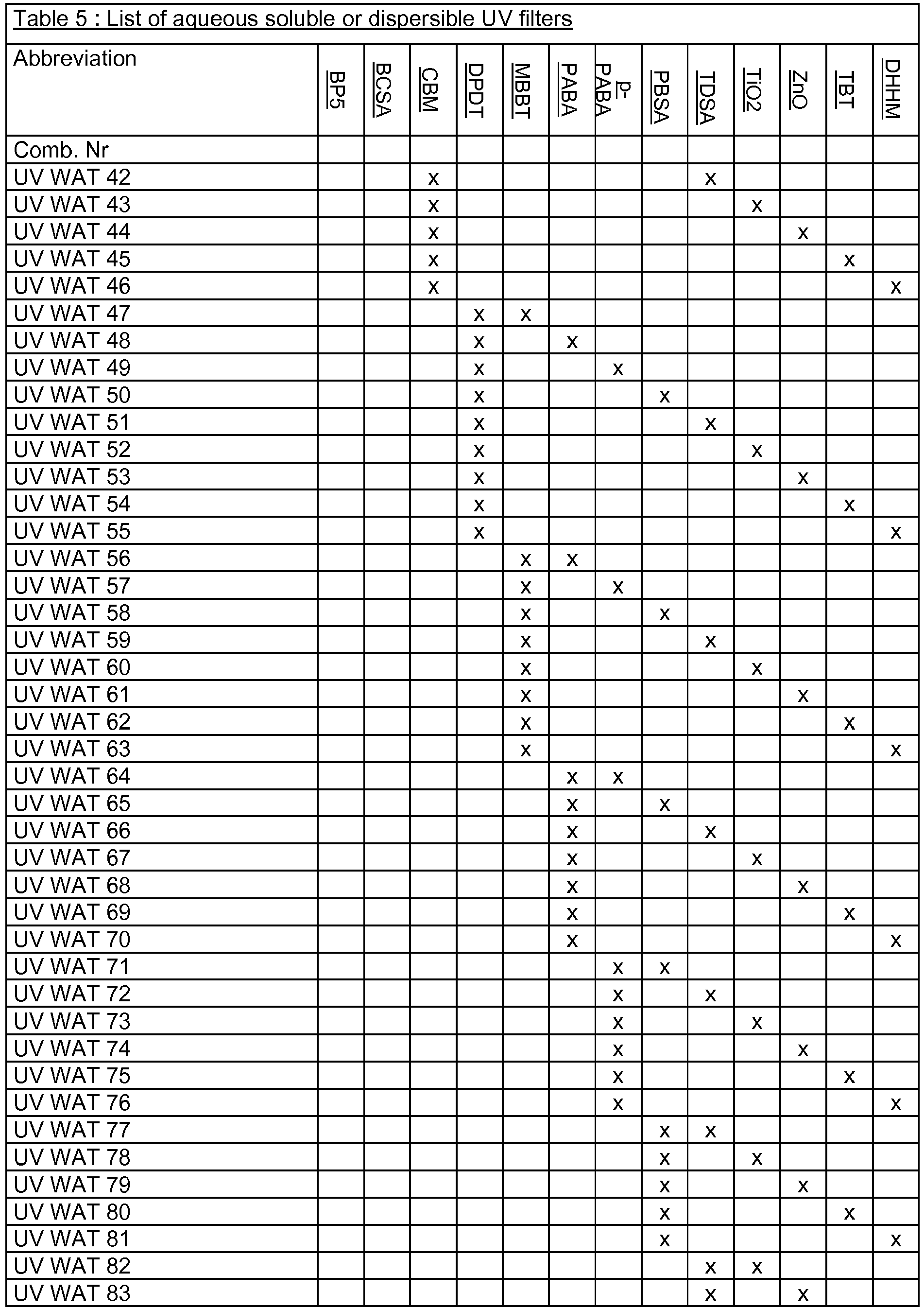

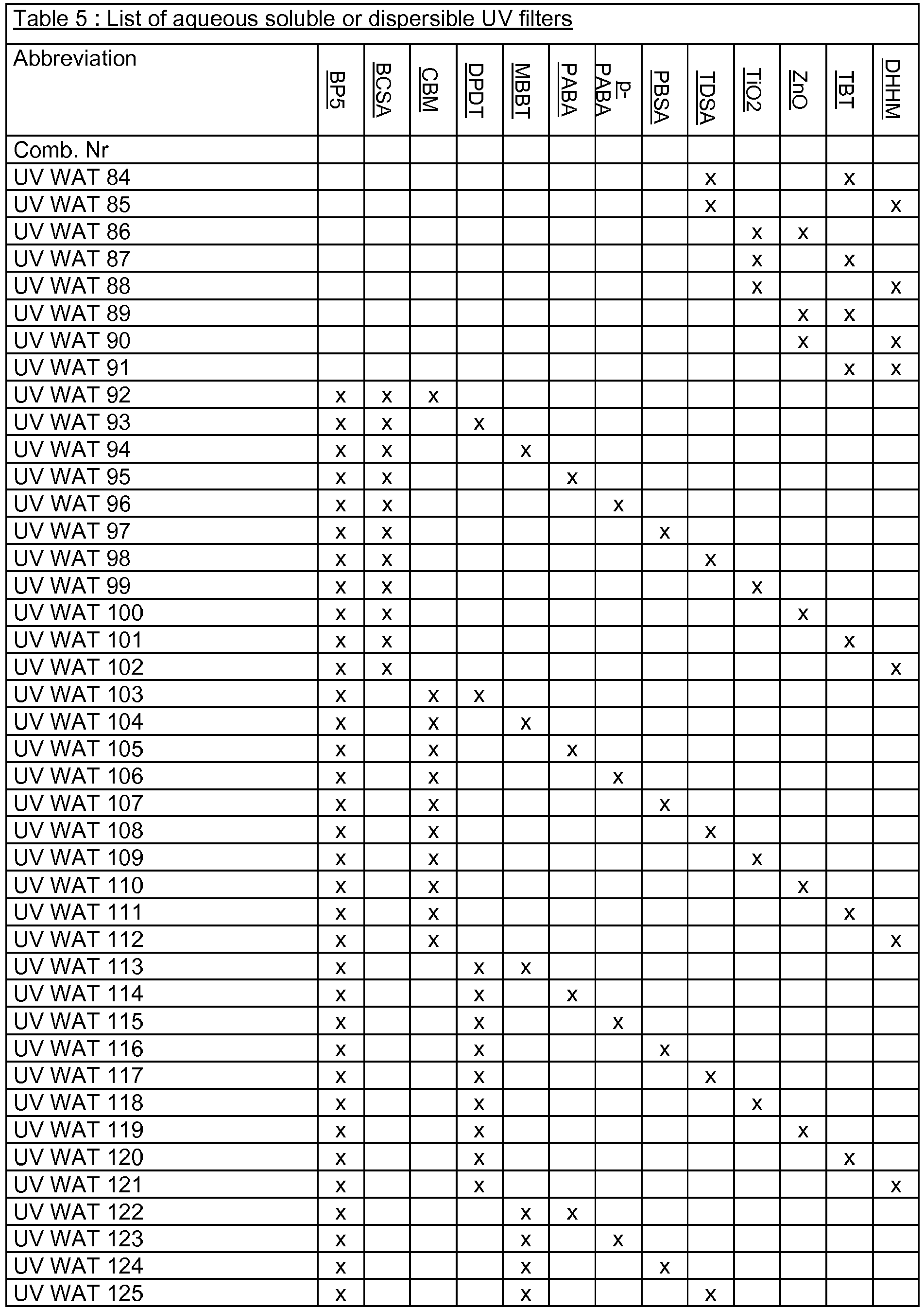

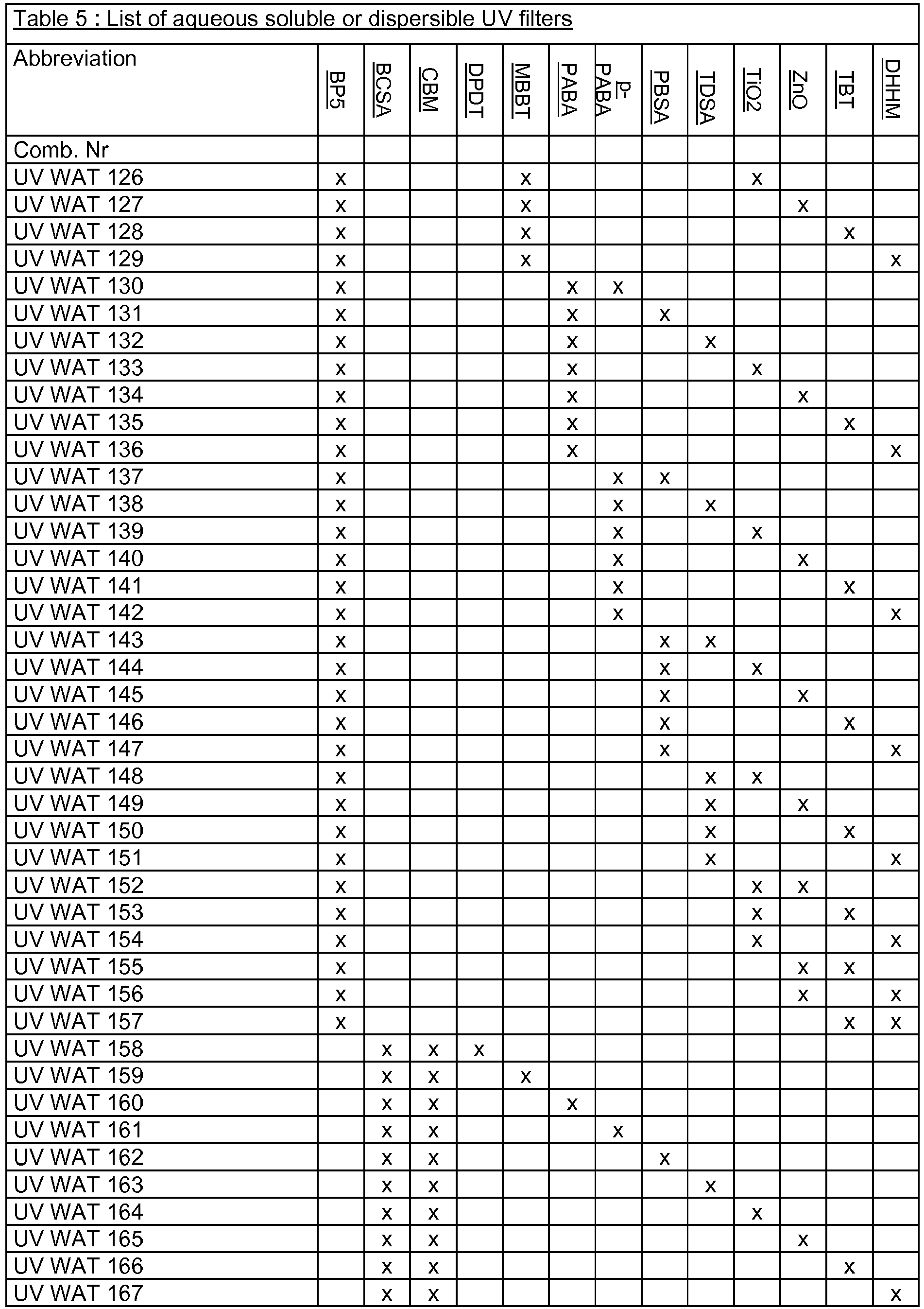

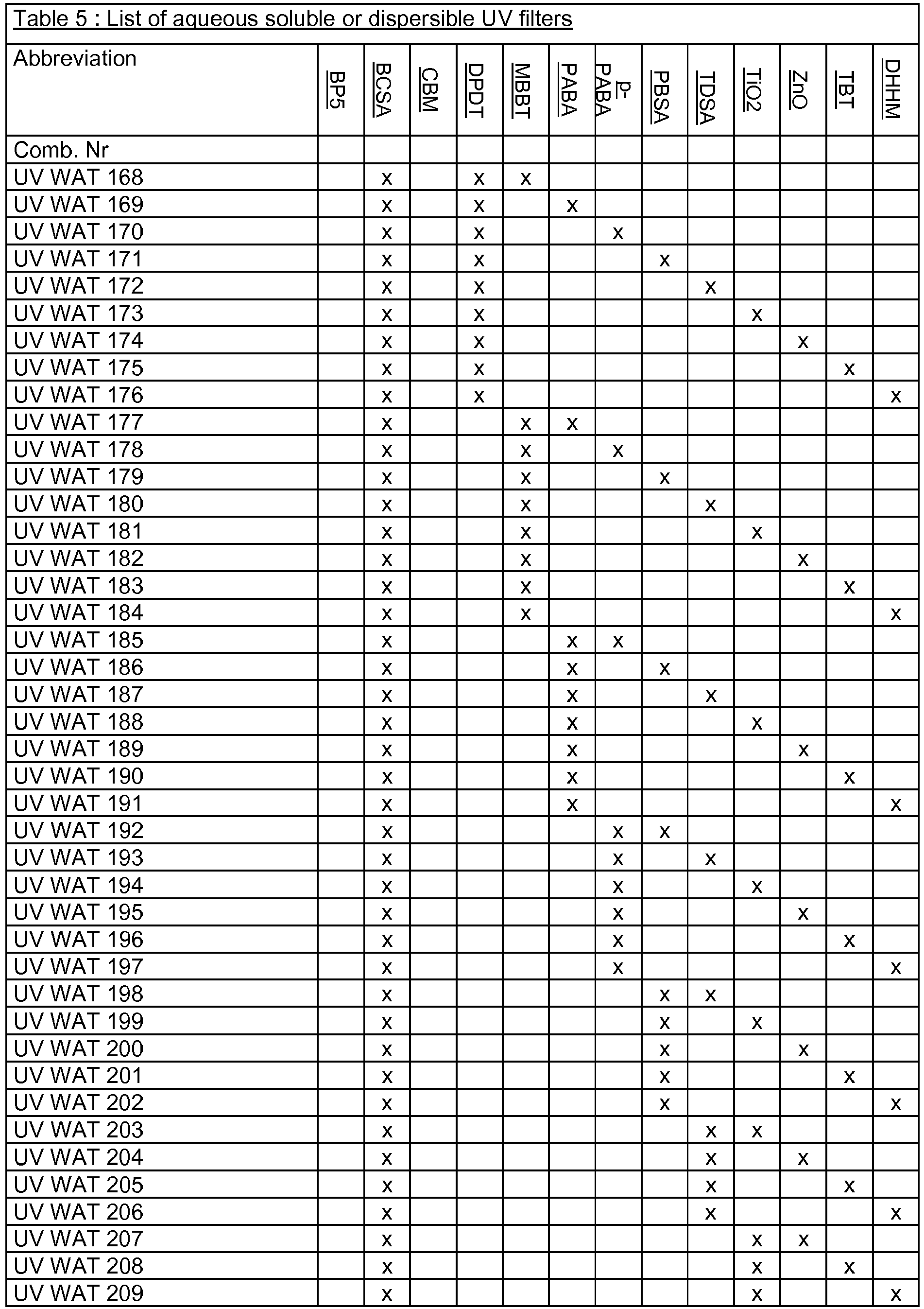

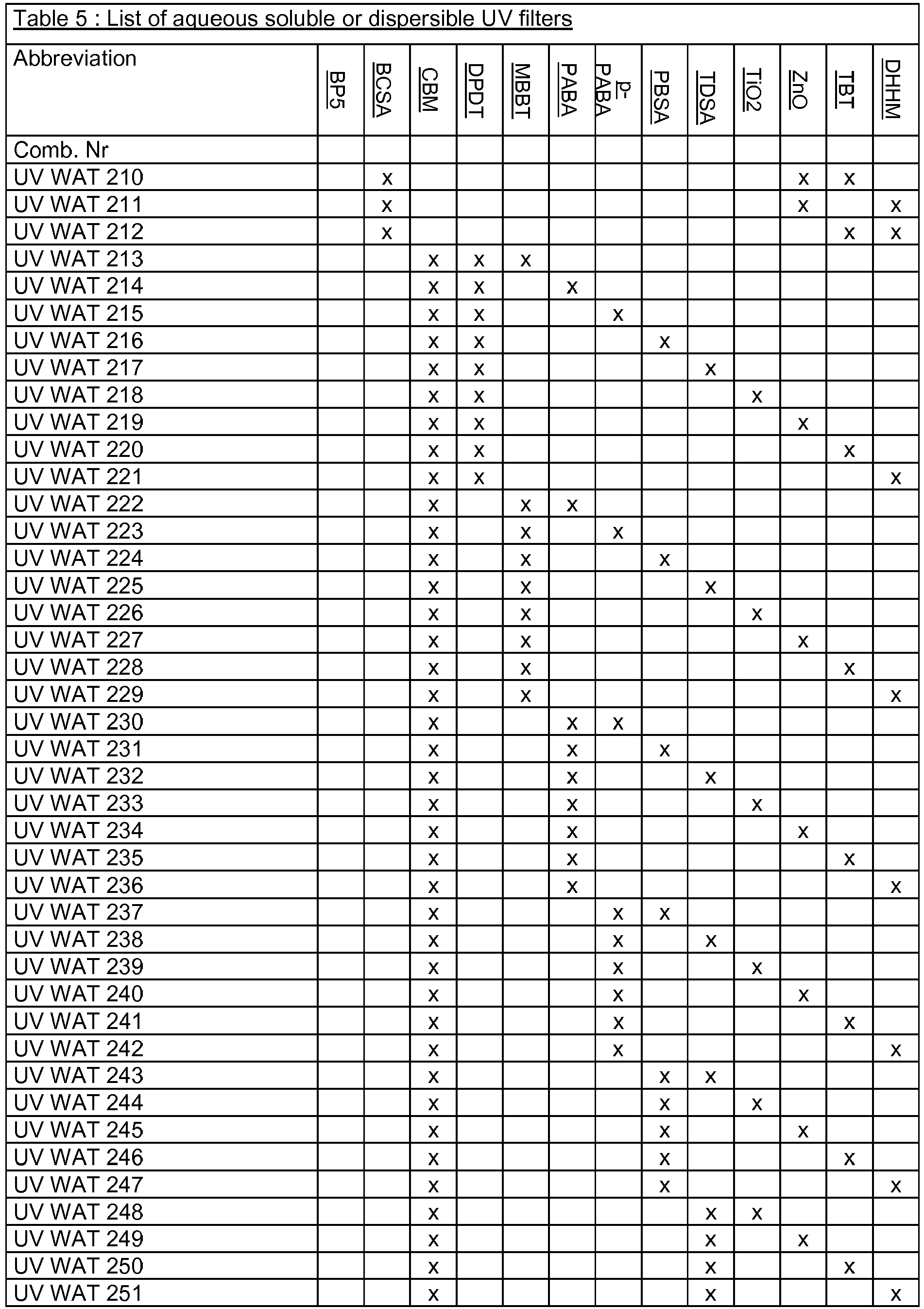

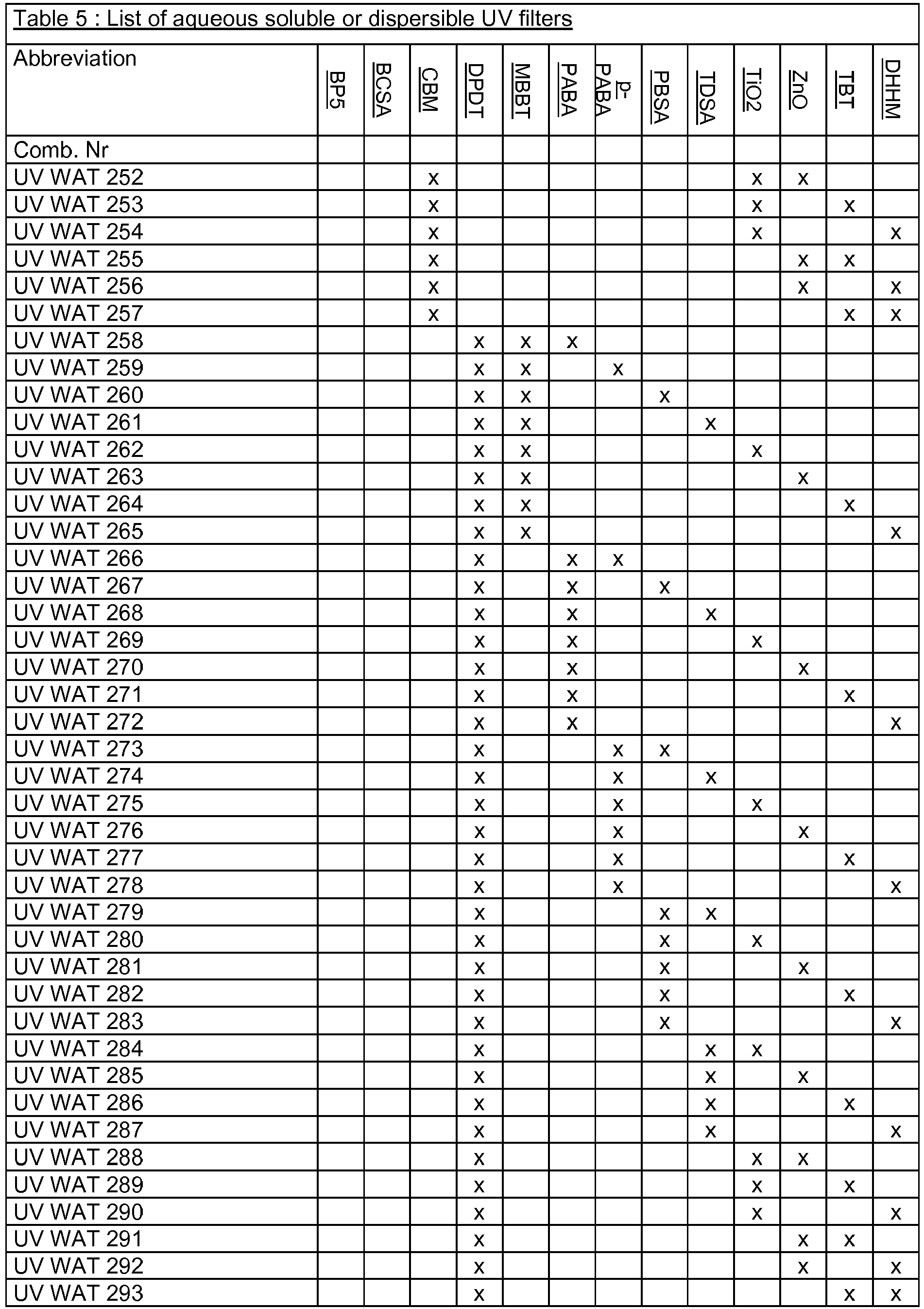

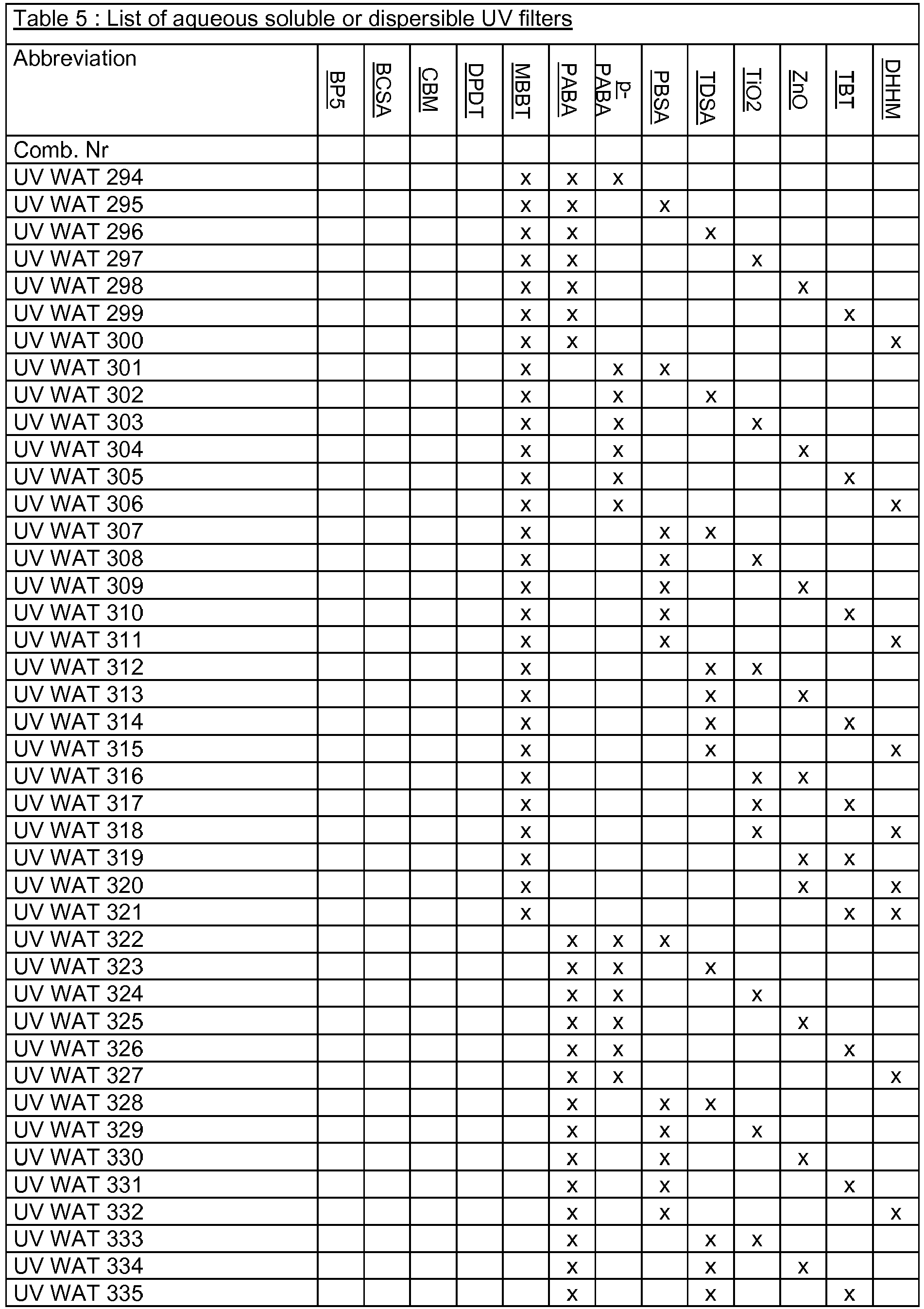

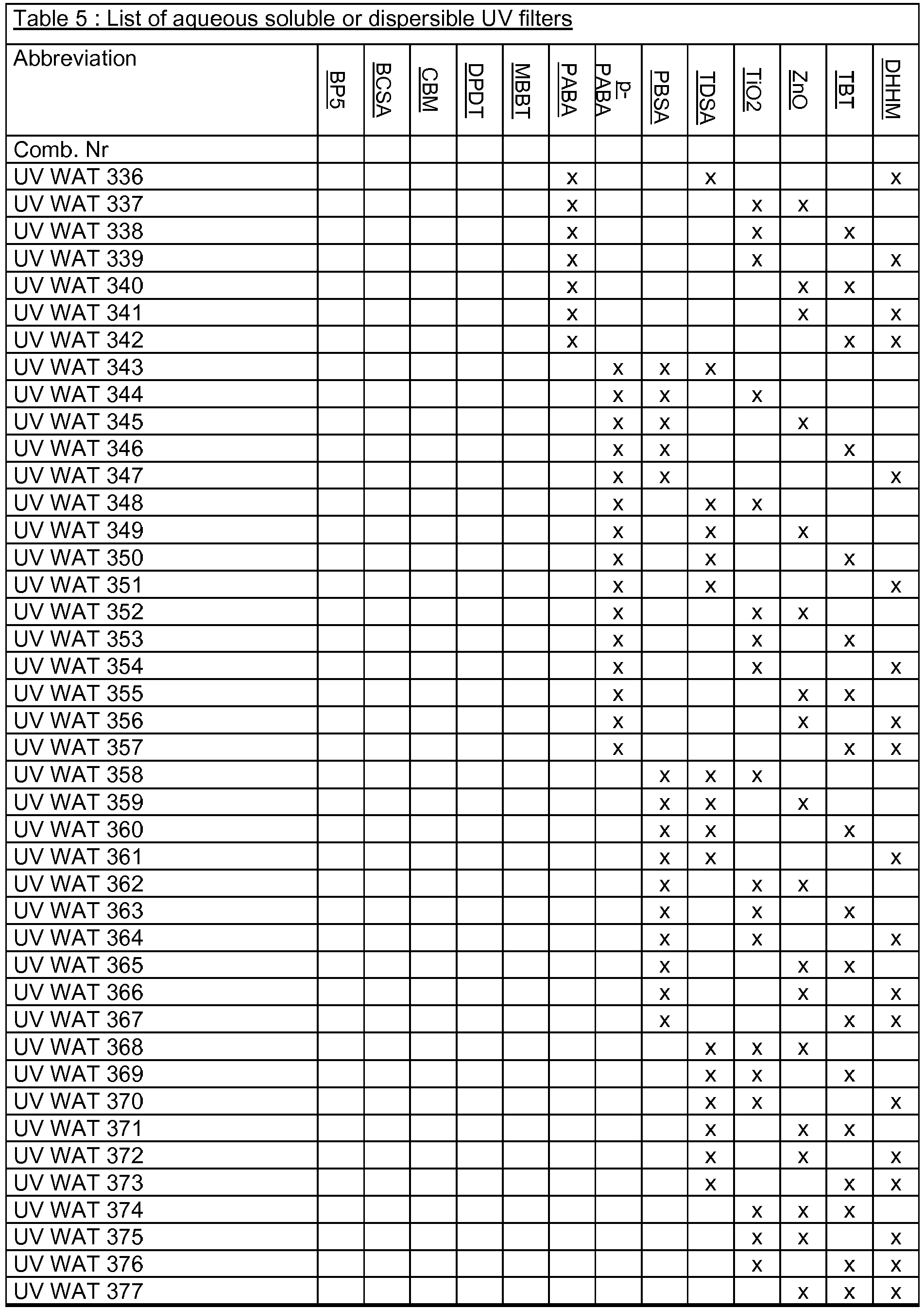

| Table 5 Abbreviations | | | |
|---|---|---|---|
| Abbreviations | INCI name | Particle size range | Cas. No. |
| BP5 | Benzophenone-5 | | 6628-37-1 |
| BCSA | Benzydilene Camphor Sulfonic Acid | | 56039-58-8 |
| CBM | Camphor Benzalkonium Methosulfate | | 52793-97-2 |
| DPDT | Disodium Phenyl Dibenzylmidazole Tetrasulfonate | | 180898-37-7 |
| MBBT | Micronized Methylene Bis-Benzotriazolyl Tatramethylbutylphenol | 50-200 nm | 103597-45-1 |
| PABA | PABA | | 150-13-0 |
| p-PABA | PEG-25 PABA | | 113010-52-9 |
| PBSA | Phenylbenzimidazole Sulfonic Acid | | 27503-81-7 |
| TDSA | Terephthalylidene Dicamphor Sulfonic Acid | | 90457-82-2 |
| TiO₂ | Titanium Dioxide | 10-50 nm | 13463-67-7 |
| ZnO | Zinc Oxide | 20-100 nm | 131413-2 |
| TB | Micronized Tris-Biphenyl Triazine | 50-200 nm | 31274-51-8 |
| DHHM | Micronized (2-{4-[2-(4-Diethylamino-2-hydroxy-benzoyl)-benzoyl]-piperazine-1-carbonyl}-phenyl)-(4-diethylamino-2-hydroxy-phenyl)-methanone | 50-200 nm | 919803-06-8 |

### UV filter combination examples

In all of the UV filter combinations listed before in the Table "X" represents a specific UV filter. The weight ratio of each specific UV absorber (based on the weight of all UV absorbers in the combination) can for example range from 0.01 to 0.99, especially 0.1 to 0.9, preferably 0.2 to 0.8. (for example 0.3).

Further UV filter combination examples are the following:
In all of the UV filter combinations listed before the weight ratio of the UV absorbers (based on the weight of all UV absorbers in the combination) is:
(a) for combinations of two UV absorbers:
   1:1 or 1:2 or 2:1 or 1:3 or 3:1.
(b) for combinations of three UV absorbers:
   1:1:1 or 1:2:1 or 1:1:2 or 2:1:1 or 1:2:2 or 2:1:2 or 2:2:1 or 1:3:1 or 1:1:3 or 3:1:1 or 1:3:3 or 3:1:3 or 3:3:1 or 1:2:3 or 1:3:2 or 2:1:3 or 2:3:1 or 3:1:2 or 3:2:1.
(c) for combinations of four UV absorbers:
   1:1:1:1 or 1:1:2:1 or 1:1:1:2 or 1:2:1:1 or 2:1:1:1 or 1:1:1:3 or 1:1:3:1
   or 1:3:1:1 or 3:1:1:1 or 1:2:2:1 or 2:1:2:1 or 2:2:1:1 or 2:1:1:2 or
   1:3:31 or 3:1:3:1 or 3:3:1:1 or 3:1:1:3 or 1:2:3:1 or 1:3:2:1 or 1:1:2:3
   or 1:1:3:2 or 2:1:1:3 or 2:1:3:1 or 2:3:1:1 or 3:1:1.2 or 3:2:1:1 or
   3:1:2:1.

### Formulation examples

In the following formulation examples the UV filter according to the present invention is MBM-09.

"UV SOL" may be (as described in Table 2) UV SOL 1, or UV SOL 2, or UV SOL 3, or UV SOL 4, or UV SOL 5, or UV SOL 6, or UV SOL 7, or UV SOL 8, or UV SOL 9, or UV SOL 10, or UV SOL 11, or UV SOL 12, or UV SOL 13, or UV SOL 14, or UV SOL 15, or UV SOL 16, or UV SOL 17, or UV SOL 18, or UV SOL 19, or UV SOL 20, or UV SOL 21, or UV SOL 22, or UV SOL 23, or UV SOL 24, or UV SOL 25, or UV SOL 26, or UV SOL 27, or UV SOL 28, or UV SOL 29, or UV SOL 30, or UV SOL 31, or UV SOL 32, or UV SOL 33, or UV SOL 34, or UV SOL 35, or UV SOL 36, or UV SOL 37, or UV SOL 38, or UV SOL 39, or UV SOL 40, or UV SOL 41, or UV SOL 42, or UV SOL 43, or UV SOL 44, or UV SOL 45, or UV SOL 46, or UV SOL 47, or UV SOL 48, or UV SOL 49, or UV SOL 50, or UV SOL 51, or UV SOL 52, or UV SOL 53, or UV SOL 54, or UV SOL 55, or UV SOL 56, or UV SOL 57, or UV SOL 58, or UV SOL 59, or UV SOL 60, or UV SOL 61, or UV SOL 62, or UV SOL 63, or UV SOL 64, or UV SOL 65, or UV SOL 66, or UV SOL 67, or UV SOL 68, or UV SOL 69, or UV SOL 70, or UV SOL 71, or UV SOL 72, or UV SOL 73, or UV SOL 74, or UV SOL 75, or UV SOL 76, or UV SOL 77, or UV SOL 78, or UV SOL 79, or UV SOL 80, or UV SOL 81, or UV SOL 82, or UV SOL 83, or UV SOL 84, or UV SOL 85, or UV SOL 86, or UV SOL 87, or UV SOL 88, or UV SOL 89, or UV SOL 90, or UV SOL 91, or UV SOL 92, or UV SOL 93, or UV SOL 94, or UV SOL 95, or UV SOL 96, or UV SOL 97, or UV SOL 98, or UV SOL 99, or UV SOL 100, or UV SOL 101, or UV SOL 102, or UV SOL 103, or UV SOL 104, or UV SOL 105, or UV SOL 106, or UV SOL 107, or UV SOL 108, or UV SOL 109, or UV SOL 110, or UV SOL 111, or UV SOL 112, or UV SOL 113, or UV SOL 114, or UV SOL 115, or UV SOL 116, or UV SOL 117, or UV SOL 118, or UV SOL 119, or UV SOL 120, or UV SOL 121, or UV SOL 122, or UV SOL 123, or UV SOL 124, or UV SOL 125, or UV SOL 126, or UV SOL 127, or UV SOL 128, or UV SOL 129, or UV SOL 130, or UV SOL 131, or UV SOL 132, or UV SOL 133, or UV SOL 134, or UV SOL 135, or UV SOL 136, or UV SOL 137, or UV SOL 138, or UV SOL 139, or UV SOL 140, or UV SOL 141, or UV SOL 142, or UV SOL 143, or UV SOL 144, or UV SOL 145, or UV SOL 146, or UV SOL 147, or UV SOL 148, or UV SOL 149, or UV SOL 150, or UV SOL 151, or UV SOL 152, or UV SOL 153, or UV SOL 154, or UV SOL 155, or UV SOL 156, or UV SOL 157, or UV SOL 158, or UV SOL 159, or UV SOL 160, or UV SOL 161, or UV SOL 162, or UV SOL 163, or UV SOL 164, or UV SOL 165, or UV SOL 166, or UV SOL 167, or UV SOL 168, or UV SOL 169, or UV SOL 170, or UV SOL 171, or UV SOL 172, or UV SOL 173, or UV SOL 174, or UV SOL 175, or UV SOL 176, or UV SOL 177, or UV SOL 178, or UV SOL 179, or UV SOL 180, or UV SOL 181, or UV SOL 182, or UV SOL 183, or UV SOL 184, or UV SOL 185, or UV SOL 186, or UV SOL 187, or UV SOL 188, or UV SOL 189, or UV SOL 190, or UV SOL 191, or UV SOL 192, or UV SOL 193, or UV SOL 194, or UV SOL 195, or UV SOL 196, or UV SOL 197, or UV SOL 198, or UV SOL 199, or UV SOL 200, or UV SOL 201, or UV SOL 202, or UV SOL 203, or UV SOL 204, or UV SOL 205, or UV SOL 206, or UV SOL 207, or UV SOL 208, or UV SOL 209, or UV SOL 210, or UV SOL 211, or UV SOL 212, or UV SOL 213, or UV SOL 214, or UV SOL 215, or UV SOL 216, or UV SOL 217, or UV SOL 218, or UV SOL 219, or UV SOL 220, or UV SOL 221, or UV SOL 222, or UV SOL 223, or UV SOL 224, or UV SOL 225, or UV SOL 226, or UV SOL 227, or UV SOL 228, or UV SOL 229, or UV SOL 230, or UV SOL 231, or UV SOL 232, or UV SOL 233, or UV SOL 234, or UV SOL 235, or UV SOL 236, or UV SOL 237, or UV SOL 238, or UV SOL 239, or UV SOL 240, or UV SOL 241, or UV SOL 242, or UV SOL 243, or UV SOL 244, or UV SOL 245, or UV SOL 246, or UV SOL 247, or UV SOL 248, or UV SOL 249, or UV SOL 250, or UV SOL 251, or UV SOL 252, or UV SOL 253;

"UV LIQ" may be (as described in Table 3) UV LIQ 1, or UV LIQ 2, or UV LIQ 3, or UV LIQ 4, or UV LIQ 5, or UV LIQ 6, or UV LIQ 7, or UV LIQ 8, or UV LIQ 9, or UV LIQ 10, or UV LIQ 11, or UV LIQ 12, or UV LIQ 13, or UV LIQ 14, or UV LIQ 15, or UV LIQ 16, or UV LIQ 17, or UV LIQ 18, or UV LIQ 19, or UV LIQ 20, or UV LIQ 21, or UV LIQ 22, or UV LIQ 23, or UV LIQ 24, or UV LIQ 25, or UV LIQ 26, or UV LIQ 27, or UV LIQ 28, or UV LIQ 29, or UV LIQ 30, or UV LIQ 31, or UV LIQ 32, or UV LIQ 33, or UV LIQ 34, or UV LIQ 35, or UV LIQ 36, or UV LIQ 37, or UV LIQ 38, or UV LIQ 39, or UV LIQ 40, or UV LIQ 41, or UV LIQ 42, or UV LIQ 43, or UV LIQ 44, or UV LIQ 45, or UV LIQ 46, or UV LIQ 47, or UV LIQ 48, or UV LIQ 49, or UV LIQ 50, or UV LIQ 51, or UV LIQ 52, or UV LIQ 53, or UV LIQ 54, or UV LIQ 55, or UV LIQ 56, or UV LIQ 57, or UV LIQ 58, or UV LIQ 59, or UV LIQ 60, or UV LIQ 61, or UV LIQ 62, or UV LIQ 63, or UV LIQ 64, or UV LIQ 65, or UV LIQ 66, or UV LIQ 67, or UV LIQ 68, or UV LIQ 69, or UV LIQ 70, or UV LIQ 71, or UV LIQ 72, or UV LIQ 73, or UV LIQ 74, or UV LIQ 75, or UV LIQ 76, or UV LIQ 77, or UV LIQ 78, or UV LIQ 79, or UV LIQ 80, or UV LIQ 81, or UV LIQ 82, or UV LIQ 83, or UV LIQ 84, or UV LIQ 85, or UV LIQ 86, or UV LIQ 87, or UV LIQ 88, or UV LIQ 89, or UV LIQ 90, or UV LIQ 91, or UV LIQ 92, or UV LIQ 93, or UV LIQ 94, or UV LIQ 95, or UV LIQ 96, or UV LIQ 97, or UV LIQ 98, or UV LIQ 99, or UV LIQ 100, or UV LIQ 101, or UV LIQ 102, or UV LIQ 103, or UV LIQ 104, or UV LIQ 105, or UV LIQ 106, or UV LIQ 107, or UV LIQ 108, or UV LIQ 109, or UV LIQ 110, or UV LIQ 111, or UV LIQ 112, or UV LIQ 113, or UV LIQ 114, or UV LIQ 115, or UV LIQ 116, or UV LIQ 117, or UV LIQ 118, or UV LIQ 119, or UV LIQ 120, or UV LIQ 121, or UV LIQ 122, or UV LIQ 123;

"UV WAT" may be (as described in Table 4) UV WAT 1, or UV WAT 2, or UV WAT 3, or UV WAT 4, or UV WAT 5, or UV WAT 6, or UV WAT 7, or UV WAT 8, or UV WAT 9, or UV WAT 10, or UV WAT 11, or UV WAT 12, or UV WAT 13, or UV WAT 14, or UV WAT 15, or UV WAT 16, or UV WAT 17, or UV WAT 18, or UV WAT 19, or UV WAT 20, or UV WAT 21, or UV WAT 22, or UV WAT 23, or UV WAT 24, or UV WAT 25, or UV WAT 26, or UV WAT 27, or UV WAT 28, or UV WAT 29, or UV WAT 30, or UV WAT 31, or UV WAT 32, or UV WAT 33, or UV WAT 34, or UV WAT 35, or UV WAT 36, or UV WAT 37, or UV WAT 38, or UV WAT 39, or UV WAT 40, or UV WAT 41, or UV WAT 42, or UV WAT 43, or UV WAT 44, or UV WAT 45, or UV WAT 46, or UV WAT 47, or UV WAT 48, or UV WAT 49, or UV WAT 50, or UV WAT 51, or UV WAT 52, or UV WAT 53, or UV WAT 54, or UV WAT 55, or UV WAT 56, or UV WAT 57, or UV WAT 58, or UV WAT 59, or UV WAT 60, or UV WAT 61, or UV WAT 62, or UV WAT 63, or UV WAT 64, or UV WAT 65, or UV WAT 66, or UV WAT 67, or UV WAT 68, or UV WAT 69, or UV WAT 70, or UV WAT 71, or UV WAT 72, or UV WAT 73, or UV WAT 74, or UV WAT 75, or UV WAT 76, or UV WAT 77, or UV WAT 78, or UV WAT 79, or UV WAT 80, or UV WAT 81, or UV WAT 82, or UV WAT 83, or UV WAT 84, or UV WAT 85, or UV WAT 86, or UV WAT 87, or UV WAT 88, or UV WAT 89, or UV WAT 90, or UV WAT 91, or UV WAT 92, or UV WAT 93, or UV WAT 94, or UV WAT 95, or UV WAT 96, or UV WAT 97, or UV WAT 98, or UV WAT 99, or UV WAT 100, or UV WAT 101, or UV WAT 102, or UV WAT 103, or UV WAT 104, or UV WAT 105, or UV WAT 106, or UV WAT 107, or UV WAT 108, or UV WAT 109, or UV WAT 110, or UV WAT 111, or UV WAT 112, or UV WAT 113, or UV WAT 114, or UV WAT 115, or UV WAT 116, or UV WAT 117, or UV WAT 118, or UV WAT 119, or UV WAT 120, or UV WAT 121, or UV WAT 122, or UV WAT 123, or UV WAT 124, or UV WAT 125, or UV WAT 126, or UV WAT 127, or UV WAT 128, or UV WAT 129, or UV WAT 130, or UV WAT 131, or UV WAT 132, or UV WAT 133, or UV WAT 134, or UV WAT 135, or UV WAT 136, or UV WAT 137, or UV WAT 138, or UV WAT 139, or UV WAT 140, or UV WAT 141, or UV WAT 142, or UV WAT 143, or UV WAT 144, or UV WAT 145, or UV WAT 146, or UV WAT 147, or UV WAT 148, or UV WAT 149, or UV WAT 150, or UV WAT 151, or UV WAT 152, or UV WAT 153, or UV WAT 154, or UV WAT 155, or UV WAT 156, or UV WAT 157, or UV WAT 158, or UV WAT 159, or UV WAT 160, or UV WAT 161, or UV WAT 162, or UV WAT 163, or UV WAT 164, or UV WAT 165, or UV WAT 166, or UV WAT 167, or UV WAT 168, or UV WAT 169, or UV WAT 170, or UV WAT 171, or UV WAT 172, or UV WAT 173, or UV WAT 174, or UV WAT 175, or UV WAT 176, or UV WAT 177, or UV WAT 178, or UV WAT 179, or UV WAT 180, or UV WAT 181, or UV WAT 182, or UV WAT 183, or UV WAT 184, or UV WAT 185, or UV WAT 186, or UV WAT 187, or UV WAT 188, or UV WAT 189, or UV WAT 190, or UV WAT 191, or UV WAT 192, or UV WAT 193, or UV WAT 194, or UV WAT 195, or UV WAT 196, or UV WAT 197, or UV WAT 198, or UV WAT 199, or UV WAT 200, or UV WAT 201, or UV WAT 202, or UV WAT 203, or UV WAT 204, or UV WAT 205, or UV WAT 206, or UV WAT 207, or UV WAT 208, or UV WAT 209, or UV WAT 210, or UV WAT 211, or UV WAT 212, or UV WAT 213, or UV WAT 214, or UV WAT 215, or UV WAT 216, or UV WAT 217, or UV WAT 218, or UV WAT 219, or UV WAT 220, or UV WAT 221, or UV WAT 222, or UV WAT 223, or UV WAT 224, or UV WAT 225, or UV WAT 226, or UV WAT 227, or UV WAT 228, or UV WAT 229, or UV WAT 230, or UV WAT 231, or UV WAT 232, or UV WAT 233, or UV WAT 234, or UV WAT 235, or UV WAT 236, or UV WAT 237, or UV WAT 238, or UV WAT 239, or UV WAT 240, or UV WAT 241, or UV WAT 242, or UV WAT 243, or UV WAT 244, or UV WAT 245, or UV WAT 246, or UV WAT 247, or UV WAT 248, or UV WAT 249, or UV WAT 250, or UV WAT 251, or UV WAT 252, or UV WAT 253, or UV WAT 254, or UV WAT 255, or UV WAT 256, or UV WAT 257, or UV WAT 258, or UV WAT 259, or UV WAT 260, or UV WAT 261, or UV WAT 262, or UV WAT 263, or UV WAT 264, or UV WAT 265, or UV WAT 266, or UV WAT 267, or UV WAT 268, or UV WAT 269, or UV WAT 270, or UV WAT 271, or UV WAT 272, or UV WAT 273, or UV WAT 274, or UV WAT 275, or UV WAT 276, or UV WAT 277, or UV WAT 278, or UV WAT 279, or UV WAT 280, or UV WAT 281, or UV WAT 282, or UV WAT 283, or UV WAT 284, or UV WAT 285, or UV WAT 286, or UV WAT 287, or UV WAT 288, or UV WAT 289, or UV WAT 290, or UV WAT 291, or UV WAT 292, or UV WAT 293, or UV WAT 294, or UV WAT 295, or UV WAT 296, or UV WAT 297, or UV WAT 298, or UV WAT 299, or UV WAT 300, or UV WAT 301, or UV WAT 302, or UV WAT 303, or UV WAT 304, or UV WAT 305, or UV WAT 306, or UV WAT 307, or UV WAT 308, or UV WAT 309, or UV WAT 310, or UV WAT 311, or UV WAT 312, or UV WAT 313, or UV WAT 314, or UV WAT 315, or UV WAT 316, or UV WAT 317, or UV WAT 318, or UV WAT 319, or UV WAT 320, or UV WAT 321, or UV WAT 322, or UV WAT 323, or UV WAT 324, or UV WAT 325, or UV WAT 326, or UV WAT 327, or UV WAT 328, or UV WAT 329, or UV WAT 330, or UV WAT 331, or UV WAT 332, or UV WAT 333, or UV WAT 334, or UV WAT 335, or UV WAT 336, or UV WAT 337, or UV WAT 338, or UV WAT 339, or UV WAT 340, or UV WAT 341, or UV WAT 342, or UV WAT 343, or UV WAT 344, or UV WAT 345, or UV WAT 346, or UV WAT 347, or UV WAT 348, or UV WAT 349, or UV WAT 350, or UV WAT 351, or UV WAT 352, or UV WAT 353, or UV WAT 354, or UV WAT 355, or UV WAT 356, or UV WAT 357, or UV WAT 358, or UV WAT 359, or UV WAT 360, or UV WAT 361, or UV WAT 362, or UV WAT 363, or UV WAT 364, or UV WAT 365, or UV WAT 366, or UV WAT 367, or UV WAT 368, or UV WAT 369, or UV WAT 370, or UV WAT 371, or UV WAT 372, or UV WAT 373, or UV WAT 374, or UV WAT 375, or UV WAT 376, or UV WAT 377.

### Formulation Examples:

| Emulsion high Protection | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Cyclomethicone | 7.0 | 7.0 | 7.0 | 7.0 | 7.0 | 7.0 | 7.0 | 7.0 | 7.0 |
| | Ethylhexyl Palmitate | 5.0 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 8.0 | 5.0 | 15.0 |
| | Glyceryl Stearate | 1.50 | 1.50 | 1.50 | 1.50 | 1.50 | 1.50 | 1.50 | 1.50 | 1.50 |
| | Potassium Cetyl Phosphate | 1.80 | 1.80 | 1.80 | 1.80 | 1.80 | 1.80 | 1.80 | 1.80 | 1.80 |
| | VP/Eicosene Copolymer | 0.60 | 0.60 | 0.60 | 0.60 | 0.60 | 0.60 | 0.60 | 0.60 | 0.60 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| Part B | Aqua | Qs to 100 | Qs to 100 | Qs to 100 | Qs to 100 | Qs to 100 | Qs to 100 | Qs to 100 | Qs to 100 | Qs to 100 |
| | Acrylates/Palmeth-25 Acrylate Copolymer | 0.25 | 0.25 | 0.25 | 0.25 | 0.25 | 0.25 | 0.25 | 0.25 | 0.25 |
| | Glycerin | 6.0 | 6.0 | 6.0 | 6.0 | 6.0 | 6.0 | 6.0 | 6.0 | 6.0 |
| | Disodium EDTA | 0.20 | 0.20 | 0.20 | 0.20 | 0.20 | 0.20 | 0.20 | 0.20 | 0.20 |
| | UV WAT | | 5.0 | 5.0 | 5.0 | 5.0 | 5.0 | 5.0 | 0.0 | 5.0 |
| Part C | Phenoxyethanol (and) Methylparaben (and) Ethylparaben (and) Butylparaben (and) Propylparaben (and) Isobutylparaben | 0.70 | 0.70 | 0.70 | 0.70 | 0.70 | 0.70 | 0.70 | 0.70 | 0.70 |
| | Tocopheryl Acetate | 0.30 | 0.30 | 0.30 | 0.30 | 0.30 | 0.30 | 0.30 | 0.30 | 0.30 |

| Sun Cream | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Cetearyl glucoside | 4.0 | 4.0 | 4.0 | 4.0 | 4.0 | 4.0 | 4.0 | 4.0 | 4.0 |
| | Dicaprylyl Carbonate | 8.0 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 8.0 | 5.0 | 15.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| Part B | Water | Qs to 100 | Qs to 100 | Qs to 100 | Qs to 100 | Qs to 100 | Qs to 100 | Qs to 100 | Qs to 100 | Qs to 100 |
| | Disodium EDTA | 0.20 | 0.20 | 0.20 | 0.20 | 0.20 | 0.20 | 0.20 | 0.20 | 0.20 |
| | Glycerin | 6.0 | 6.0 | 6.0 | 6.0 | 6.0 | 6.0 | 6.0 | 6.0 | 6.0 |
| | PVP/dimethylconylacrylate/polycarbamyl/polyglycol ester | 3.0 | 3.0 | 3.0 | 3.0 | 3.0 | 3.0 | 3.0 | 3.0 | 3.0 |
| | UV WAT | | 5.0 | 5.0 | 5.0 | 5.0 | 5.0 | 5.0 | 0.0 | 5.0 |
| | Sodium polyacrylate | 0.70 | 0.70 | 0.70 | 0.70 | 0.70 | 0.70 | 0.70 | 0.70 | 0.70 |
| Part C | Dimethicone | 3.0 | 3.0 | 3.0 | 3.0 | 3.0 | 3.0 | 3.0 | 3.0 | 3.0 |
| | Corn Starch modified | 3.0 | 3.0 | 3.0 | 3.0 | 3.0 | 3.0 | 3.0 | 3.0 | 3.0 |
| Part E | Phenoxyethanol (and) Methylparaben (and) Ethylparaben (and) Butylparaben (and) Propylparaben (and) Isobutylparaben | 1.0 | 1.0 | 1.0 | 1.0 | 1.0 | 1.0 | 1.0 | 1.0 | 1.0 |

| Sunscreen Gel | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Alcohol Denatured | Qs to 100 | Qs to 100 | Qs to 100 | Qs to 100 | Qs to 100 | Qs to 100 | Qs to 100 | Qs to 100 | Qs to 100 |
| | Hydroxypropyl Cellulose | 1.0 | 1.0 | 1.0 | 1.0 | 1.0 | 1.0 | 1.0 | 1.0 | 1.0 |
| | Acrylates/Octylacrylamide Copolymer | 1.0 | 1.0 | 1.0 | 1.0 | 1.0 | 1.0 | 1.0 | 1.0 | 1.0 |
| | C12-15 Alkyl Benzoate | 10.0 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 8.0 | 5.0 | 15.0 |
| | Cyclotetrasiloxane (and) Cyclopentasiloxane | 3.0 | 3.0 | 3.0 | 3.0 | 3.0 | 3.0 | 3.0 | 3.0 | 3.0 |
| | PEG/PPG-4/12 Dimethicone | 1.0 | 1.0 | 1.0 | 1.0 | 1.0 | 1.0 | 1.0 | 1.0 | 1.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | UV WAT | | 5.0 | 5.0 | 5.0 | 5.0 | 5.0 | 5.0 | 0.0 | 5.0 |

| Gel Cream | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Sodium Carbomer | 0.50 | 0.50 | 0.50 | 0.50 | 0.50 | 0.50 | 0.50 | 0.50 | 0.50 |
| | Acrylates/C10-C30 Alkyl Acrylate | 1.0 | 1.0 | 1.0 | 1.0 | 1.0 | 1.0 | 1.0 | 1.0 | 1.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | UV WAT | | 5.0 | 5.0 | 5.0 | 5.0 | 5.0 | 5.0 | 0.0 | 5.0 |
| | C12-15 Alkyl Benzoate | 2.0 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 8.0 | 5.0 | 15.0 |
| | Butylenglycol Dicaprylat/Dicaprate | 4.0 | 0.0 | 0.0 | 0.0 | 0.0 | 0.0 | 0.0 | 0.0 | 0.0 |
| | Cetyl Dimethicone | 2.0 | 2.0 | 2.0 | 2.0 | 2.0 | 2.0 | 2.0 | 2.0 | 2.0 |
| | Glycerine | 0.50 | 0.50 | 0.50 | 0.50 | 0.50 | 0.50 | 0.50 | 0.50 | 0.50 |
| | Tocopherol | 3.0 | 3.0 | 3.0 | 3.0 | 3.0 | 3.0 | 3.0 | 3.0 | 3.0 |
| | Methylparabene | 1.0 | 1.0 | 1.0 | 1.0 | 1.0 | 1.0 | 1.0 | 1.0 | 1.0 |
| | Phenoxyethanol | 1.0 | 1.0 | 1.0 | 1.0 | 1.0 | 1.0 | 1.0 | 1.0 | 1.0 |
| | Water | ad 100 | ad 100 | ad 100 | ad 100 | ad 100 | ad 100 | ad 100 | ad 100 | ad 100 |

| Emulsifier Free | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Acrylates/C10-30 Alkyl Acrylate Crosspolymer | 0.40 | 0.40 | 0.40 | 0.40 | 0.40 | 0.40 | 0.40 | 0.40 | 0.40 |
| | Xanthan Gum | 0.10 | 0.10 | 0.10 | 0.10 | 0.10 | 0.10 | 0.10 | 0.10 | 0.10 |
| | UV SOL | | 10.0 | 15.0 | 5.0 | 10.0 | 5.0 | 8.0 | 5.0 | 15.0 |
| | UV LIQ | | 5.0 | 10.0 | 5.0 | 10.0 | 5.0 | 10.0 | 5.0 | 5.0 |
| | UV WAT | | 5.0 | 5.0 | 10.0 | 10.0 | 15.0 | 15.0 | 15.0 | 0.0 |
| | UV filter according to the present invention | | 3.0 | 5.0 | 5.0 | 5.0 | 5.0 | 5.0 | 0.0 | 5.0 |
| | C12-15 Alkyl Benzoate | 6.0 | 0.0 | 0.0 | 0.0 | 0.0 | 0.0 | 0.0 | 0.0 | 0.0 |
| | Octyldodecanol | 7.0 | 0.0 | 0.0 | 0.0 | 0.0 | 0.0 | 0.0 | 0.0 | 0.0 |
| | Cetyl Dimethicone | 6.0 | 6.0 | 6.0 | 6.0 | 6.0 | 6.0 | 6.0 | 6.0 | 6.0 |
| | Ethylhexyloxyglycerine | 1.0 | 1.0 | 1.0 | 1.0 | 1.0 | 1.0 | 1.0 | 1.0 | 1.0 |
| | Butylen Glycol | 10.0 | 10.0 | 10.0 | 10.0 | 10.0 | 10.0 | 10.0 | 10.0 | 10.0 |
| | Glycinc Soja | 1.0 | 1.0 | 1.0 | 1.0 | 1.0 | 1.0 | 1.0 | 1.0 | 1.0 |
| | Vitamin E Acetatc | 0.25 | 0.25 | 0.25 | 0.25 | 0.25 | 0.25 | 0.25 | 0.25 | 0.25 |
| | Trisodium EDTA | 0.10 | 0.10 | 0.10 | 0.10 | 0.10 | 0.10 | 0.10 | 0.10 | 0.10 |
| | Ethanol | 3.50 | 3.50 | 3.50 | 3.50 | 3.50 | 3.50 | 3.50 | 3.50 | 3.50 |
| | Parfume | 0.20 | 0.20 | 0.20 | 0.20 | 0.20 | 0.20 | 0.20 | 0.20 | 0.20 |
| | Watersoluble Dyes | 0.02 | 0.02 | 0.02 | 0.02 | 0.02 | 0.02 | 0.02 | 0.02 | 0.02 |
| | Water | ad 100 | ad 100 | ad 100 | ad 100 | ad 100 | ad 100 | ad 100 | ad 100 | ad 100 |

| Sun Spray | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Ethyl Trisiloxane | 2.50 | 2.50 | 2.50 | 2.50 | 2.50 | 2.50 | 2.50 | 2.50 | 2.50 |
| | Hydrogenated Cocoglycerides | 1.50 | 1.50 | 1.50 | 1.50 | 1.50 | 1.50 | 1.50 | 1.50 | 1.50 |
| | C12-15 Alkyl Benzoate | 3.60 | 3.60 | 3.60 | 3.60 | 3.60 | 3.60 | 3.60 | 3.60 | 3.60 |
| | UV filter according to the present invention | | 5.0 | 3.0 | 5.0 | 5.0 | 5.0 | 5.0 | 0.0 | 5.0 |
| | UV SOL | | 10.0 | 15.0 | 5.0 | 10.0 | 5.0 | 8.0 | 5.0 | 15.0 |
| | UV LIQ | | 5.0 | 10.0 | 5.0 | 10.0 | 5.0 | 10.0 | 5.0 | 5.0 |
| Part B | Water (and) Caprylic/Capric Triglyceride (and) Glycerin (and) Ceteareth-25 (and) Disodium Ethylene Di(Cocamide PEG-15 Disulfate) (and) Sodium Lauroyl Lactylate (and) Behenyl Alcohol (and) Glyceryl Stearate (and) Glyceryl Stearate Citrate (and) Xanthan Gum | 10.0 | 10.0 | 10.0 | 10.0 | 10.0 | 10.0 | 10.0 | 10.0 | 10.0 |
| | PVP/Hexadecene Copolymer | 0.50 | 0.50 | 0.50 | 0.50 | 0.50 | 0.50 | 0.50 | 0.50 | 0.50 |
| | Aqua | Qs to 10 | Qs to 10 | Qs to 10 | Qs to 10 | Qs to 10 | Qs to 10 | Qs to 10 | Qs to 10 | Qs to 10 |
| | Glycerin | 3.0 | 3.0 | 3.0 | 3.0 | 3.0 | 3.0 | 3.0 | 3.0 | 3.0 |
| | UV WAT | | 5.0 | 5.0 | 10.0 | 10.0 | 15.0 | 15.0 | 15.0 | 0.0 |
| Part C | Alcohol Denatured | 7.0 | 7.0 | 7.0 | 7.0 | 7.0 | 7.0 | 7.0 | 7.0 | 7.0 |
| Part D | Phenoxyethanol (and) Methylparaben (and) Ethylparaben (and) Butylparaben (and) Propylparaben (and) Isobutylparaben | 0.70 | 0.70 | 0.70 | 0.70 | 0.70 | 0.70 | 0.70 | 0.70 | 0.70 |
| | Tocopheryl Acetate | 0.60 | 0.60 | 0.60 | 0.60 | 0.60 | 0.60 | 0.60 | 0.60 | 0.60 |

| Sun spray foaming | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Behenyl Alcohol (and) Glyceryl Stearate (and) Glyceryl Stearate Citrate (and) Disodium Ethylene Di(Cocamide PEG-15 Disulfate) | 0.50 | 0.50 | 0.50 | 0.50 | 0.50 | 0.50 | 0.50 | 0.50 | 0.50 |
| | Isotrideceth-12 | 0.50 | 0.50 | 0.50 | 0.50 | 0.50 | 0.50 | 0.50 | 0.50 | 0.50 |
| | Hydrogenated Cocoglycerides | 1.50 | 0.0 | 0.0 | 0.0 | 0.0 | 0.0 | 0.0 | 0.0 | 0.0 |
| | C12-15 Alkyl Benzoate | 8.0 | 0.0 | 0.0 | 0.0 | 0.0 | 0.0 | 0.0 | 0.0 | 0.0 |
| | UV filter according to the present invention | | 5.0 | 5.0 | 5.0 | 5.0 | 5.0 | 5.0 | 0.0 | 5.0 |
| | UV SOL | | 3.0 | 15.0 | 5.0 | 10.0 | 5.0 | 8.0 | 5.0 | 15.0 |
| | UV LIQ | | 5.0 | 10.0 | 5.0 | 10.0 | 5.0 | 10.0 | 5.0 | 5.0 |
| Part B | Aqua | 5.0 | 5.0 | 5.0 | 5.0 | 5.0 | 5.0 | 5.0 | 5.0 | 5.0 |
| | Glycerin | 4.0 | 4.0 | 4.0 | 4.0 | 4.0 | 4.0 | 4.0 | 4.0 | 4.0 |
| | Galactoarabinan | 0.25 | 0.25 | 0.25 | 0.25 | 0.25 | 0.25 | 0.25 | 0.25 | 0.25 |
| | UV WAT | | 5.0 | 5.0 | 10.0 | 10.0 | 15.0 | 15.0 | 15.0 | 0.0 |
| Part C | Disodium Ethylene Di(Cocamide PEG-15 Disulfate) (and) Sodium Lauroyl Lactylate | 1.0 | 1.0 | 1.0 | 1.0 | 1.0 | 1.0 | 1.0 | 1.0 | 1.0 |
| | Water | Qs to 100 | Qs to 100 | Qs to 100 | Qs to 100 | Qs to 100 | Qs to 100 | Qs to 100 | Qs to 100 | Qs to 100 |
| Part D | Phenoxyethanol (and) Methylparaben (and) Ethylparaben (and) Butylparaben (and) Propylparaben (and) Isobutylparaben | 0.70 | 0.70 | 0.70 | 0.70 | 0.70 | 0.70 | 0.70 | 0.70 | 0.70 |
| | Tocopheryl Acetate | 0.60 | 0.60 | 0.60 | 0.60 | 0.60 | 0.60 | 0.60 | 0.60 | 0.60 |

| Active Naturals Continuous Spray | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Acrylates/Octylacrylamide Copolymer | 3,0 | 3,01 | 3,02 | 3,03 | 3,04 | 3,05 | 3,06 | 3,07 | 3,08 |
| | Ascorbyl Palmitate | 0,30 | 0,31 | 0,32 | 0,33 | 0,34 | 0,35 | 0,36 | 0,37 | 0,38 |
| | Diisopropyl Adipate | 3,0 | 0.0 | 0.0 | 0.0 | 0.0 | 0.0 | 0.0 | 0.0 | 0.0 |
| | Glycerine soja seed extract soybean | 3,0 | 0.0 | 0.0 | 0.0 | 0.0 | 0.0 | 0.0 | 0.0 | 0.0 |
| | Isodecyl Neopentanoate | 2,50 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 8.0 | 5.0 | 15.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | UV WAT | | 5.0 | 5.0 | 5.0 | 5.0 | 5.0 | 5.0 | 0.0 | 5.0 |
| | Lauryl Lactate | 1,50 | 1,50 | 1,50 | 1,50 | 1,50 | 1,50 | 1,50 | 1,50 | 1,50 |
| | PPG-12/SMDI Copolymer | 1,0 | 1,0 | 1,0 | 1,0 | 1,0 | 1,0 | 1,0 | 1,0 | 1,0 |
| | Retinyl Palmitate | 0,30 | 0,30 | 0,30 | 0,30 | 0,30 | 0,30 | 0,30 | 0,30 | 0,30 |
| | SD Alcohol 40 | 80% V/V | 80% V/V | 80% V/V | 80% V/V | 80% V/V | 80% V/V | 80% V/V | 80% V/V | 80% V/V |
| | Tocopherol Acetate | 1,0 | 1,0 | 1,0 | 1,0 | 1,0 | 1,0 | 1,0 | 1,0 | 1,0 |
| | Parfume | q.s. | q.s. | q.s. | q.s. | q.s. | q.s. | q.s. | q.s. | q.s. |
| | Aqua | ad 100 | ad 100 | ad 100 | ad 100 | ad 100 | ad 100 | ad 100 | ad 100 | ad 100 |
| | Propellent | Qs | Qs | Qs | Qs | Qs | Qs | Qs | Qs | Qs |

| W/O Sunscreen Lotion | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | PEG-7 Hydrogenated Castor Oil | 3.0 | 3.0 | 3.0 | 3.0 | 3.0 | 3.0 | 3.0 | 3.0 | 3.0 |
| | Polyglyceryl-3 Diisostearate | 4.0 | 4.0 | 4.0 | 4.0 | 4.0 | 4.0 | 4.0 | 4.0 | 4.0 |
| | Microcrystalline Wax | 1.0 | 1.0 | 1.0 | 1.0 | 1.0 | 1.0 | 1.0 | 1.0 | 1.0 |
| | Magnesium Stearate | 1.50 | 1.50 | 1.50 | 1.50 | 1.50 | 1.50 | 1.50 | 1.50 | 1.50 |
| | Propylparaben | 0.10 | 0.10 | 0.10 | 0.10 | 0.10 | 0.10 | 0.10 | 0.10 | 0.10 |
| | Mineral Oil | 15.0 | 5.0 | 0.0 | 10.0 | 0.0 | 5.0 | 0.0 | 5.0 | 10.0 |
| | UV filter according to the present invention | | 3.0 | 15.0 | 5.0 | 10.0 | 5.0 | 5.0 | 5.0 | 15.0 |
| | Octyldodecanol | 8.0 | 0.0 | 0.0 | 0.0 | 0.0 | 0.0 | 0.0 | 0.0 | 0.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| Part B | Water | qs to 100 | qs to 100 | qs to 100 | qs to 100 | qs to 100 | qs to 100 | qs to 100 | qs to 100 | qs to 100 |
| | UV WAT | | 5.0 | 5.0 | 5.0 | 5.0 | 5.0 | 5.0 | 0.0 | 5.0 |
| Part C | Water (and) Citric Acid | 0.05 | 0.05 | 0.05 | 0.05 | 0.05 | 0.05 | 0.05 | 0.05 | 0.05 |
| | Methylparaben | 0.15 | 0.15 | 0.15 | 0.15 | 0.15 | 0.15 | 0.15 | 0.15 | 0.15 |
| | Magnesium Sulfate | 0.50 | 0.50 | 0.50 | 0.50 | 0.50 | 0.50 | 0.50 | 0.50 | 0.50 |
| | Fragrance | qs | qs | qs | qs | qs | qs | qs | qs | qs |

| W/Si sun cream | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Cyclopentasiloxane (and) Dimethicone/Vinyl Dimethicone Crosspolymer | 3.0 | 3.0 | 3.0 | 3.0 | 3.0 | 3.0 | 3.0 | 3.0 | 3.0 |
| | PEG-10 Dimethicone | 0.30 | 0.30 | 0.30 | 0.30 | 0.30 | 0.30 | 0.30 | 0.30 | 0.30 |
| | Polyglyceryl-3 Polydimethylsiloxyethyl Dimethicone | 4.0 | 4.0 | 4.0 | 4.0 | 4.0 | 4.0 | 4.0 | 4.0 | 4.0 |
| | Dimethicone | 5.0 | 5.0 | 5.0 | 5.0 | 5.0 | 5.0 | 5.0 | 5.0 | 5.0 |
| | Cyclomethicone | 15.0 | 15.0 | 15.0 | 15.0 | 15.0 | 15.0 | 15.0 | 15.0 | 15.0 |
| | UV filter according to the present invention | | 3.0 | 15.0 | 5.0 | 10.0 | 5.0 | 5.0 | 5.0 | 15.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| Part C | 1.3-Butylen Glycol | 8.0 | 8.0 | 8.0 | 8.0 | 8.0 | 8.0 | 8.0 | 8.0 | 8.0 |
| | Sodium Citrate | 0.20 | 0.20 | 0.20 | 0.20 | 0.20 | 0.20 | 0.20 | 0.20 | 0.20 |
| | Ethyl Alcohol | 5.0 | 5.0 | 5.0 | 5.0 | 5.0 | 5.0 | 5.0 | 5.0 | 5.0 |
| | Sodium Chloride | 0.50 | 0.50 | 0.50 | 0.50 | 0.50 | 0.50 | 0.50 | 0.50 | 0.50 |
| | UV WAT | | 5.0 | 5.0 | 5.0 | 5.0 | 5.0 | 5.0 | 0.0 | 5.0 |
| | Aqua | Qs to 100 | Qs to 100 | Qs to 100 | Qs to 100 | Qs to 100 | Qs to 100 | Qs to 100 | Qs to 100 | Qs to 100 |

| Lipstick | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Caprylic/Capric Triglyceride | 12.0 0 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 5.0 | 5.0 | 15.0 |
| | Octyldodecanol | 7.0 | 7.0 | 7.0 | 7.0 | 7.0 | 7.0 | 7.0 | 7.0 | 7.0 |
| | Pentaerythrityl Tetraisostearate | 10.0 | 5.0 | 0.0 | 5.0 | 0.0 | 0.0 | 0.0 | 0.0 | 5.0 |
| | Polyglyceryl-3 Diisostearate | 2.50 | 2.50 | 2.50 | 2.50 | 2.50 | 2.50 | 2.50 | 2.50 | 2.50 |
| | Bis-Diglyceryl Polyacyladipate-2 | 9.0 | 9.0 | 9.0 | 9.0 | 9.0 | 9.0 | 9.0 | 9.0 | 9.0 |
| | Cetearyl Alcohol | 8.0 | 8.0 | 8.0 | 8.0 | 8.0 | 8.0 | 8.0 | 8.0 | 8.0 |
| | Myristyl Myristate | 3.50 | 3.50 | 3.50 | 3.50 | 3.50 | 3.50 | 3.50 | 3.50 | 3.50 |
| | Beeswax | 5.0 | 5.0 | 5.0 | 5.0 | 5.0 | 5.0 | 5.0 | 5.0 | 5.0 |
| | Copernicia Cerifera (Carnauba) Wax | 1.50 | 1.50 | 1.50 | 1.50 | 1.50 | 1.50 | 1.50 | 1.50 | 1.50 |
| | Cera Alba | 0.50 | 0.50 | 0.50 | 0.50 | 0.50 | 0.50 | 0.50 | 0.50 | 0.50 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | Tocopheryl Acetate | 0.50 | 0.50 | 0.50 | 0.50 | 0.50 | 0.50 | 0.50 | 0.50 | 0.50 |
| | Tocopherol; Ascorbyl Palmitate | 0.05 | 0.05 | 0.05 | 0.05 | 0.05 | 0.05 | 0.05 | 0.05 | 0.05 |
| | Simmondsia Chinensis (Jojoba) Seed Extract | 2.0 | 2.0 | 2.0 | 2.0 | 2.0 | 2.0 | 2.0 | 2.0 | 2.0 |
| | Parfum. BHT | q.s | q.s | q.s | q.s | q.s | q.s | q.s | q.s | q.s |
| | Ricinus Communis | QS to 100 | QS to 100 | QS to 100 | QS to 100 | QS to 100 | QS to 100 | QS to 100 | QS to 100 | QS to 100 |

| Waterproof Gel | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Anhydrous Ethanol | Qsp 100 | Qsp 100 | Qsp 100 | Qsp 100 | Qsp 100 | Qsp 100 | Qsp 100 | Qsp 100 | Qsp 100 |
| | Hydroxypropyl Cellulose | 1.0 | 1.0 | 1.0 | 1.0 | 1.0 | 1.0 | 1.0 | 1.0 | 1.0 |
| | Acrylates/Octylacrylamide Copolymer | 1.0 | 1.0 | 1.0 | 1.0 | 1.0 | 1.0 | 1.0 | 1.0 | 1.0 |
| | UV filter according to the present invention | | 3.0 | 15.0 | 5.0 | 10.0 | 5.0 | 5.0 | 5.0 | 15.0 |
| | C12-15 Alkyl Benzoate | 10.0 | 10.0 | 10.0 | 10.0 | 10.0 | 10.0 | 10.0 | 10.0 | 10.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | Cyclomethicone | 3.0 | 3.0 | 3.0 | 3.0 | 3.0 | 3.0 | 3.0 | 3.0 | 3.0 |
| | PEG/PPG-4/12 Dimethicone | 1.0 | 1.0 | 1.0 | 1.0 | 1.0 | 1.0 | 1.0 | 1.0 | 1.0 |

| SUNSCREEN Oleogel | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Isopropyl Myristate | 38.0 | qs. 100 | qs. 100 | qs. 100 | qs. 100 | qs. 100 | qs. 100 | qs. 100 | qs. 100 |
| | C12-15 Alkyl Benzoate | 10.0 | 5.0 | 5.0 | 5.0 | 5.0 | 5.0 | 5.0 | 5.0 | 5.0 |
| | UV filter according to the present invention | | 3.0 | 15.0 | 5.0 | 10.0 | 5.0 | 5.0 | 5.0 | 15.0 |
| | Caprylic/Capric Triglyceride | 39.0 | 15.0 | 15.0 | 15.0 | 15.0 | 15.0 | 15.0 | 15.0 | 15.0 |
| | Perfume | q.s. | q.s. | q.s. | q.s. | q.s. | q.s. | q.s. | q.s. | q.s. |
| | Disteardimonium Hectorite | 15.0 | 15.0 | 15.0 | 15.0 | 15.0 | 15.0 | 15.0 | 15.0 | 15.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | Propylene Carbonate | 5.0 | 5.0 | 5.0 | 5.0 | 5.0 | 5.0 | 5.0 | 5.0 | 5.0 |

| O/W/O Soft Cream | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | PEG-60 Hydrogenated Castor Oil | 0.40 | 0.40 | 0.40 | 0.40 | 0.40 | 0.40 | 0.40 | 0.40 | 0.40 |
| | Water | 0.90 | 0.90 | 0.90 | 0.90 | 0.90 | 0.90 | 0.90 | 0.90 | 0.90 |
| Part B | Tocopheryl Acetate | 2.0 | 2.0 | 2.0 | 2.0 | 2.0 | 2.0 | 2.0 | 2.0 | 2.0 |
| | Retinyl Palmitate | 0.50 | 0.50 | 0.50 | 0.50 | 0.50 | 0.50 | 0.50 | 0.50 | 0.50 |
| | Caprylic/Capric Triglyceride | 2.50 | 2.50 | 2.50 | 2.50 | 2.50 | 2.50 | 2.50 | 2.50 | 2.50 |
| Part C | Water | Qs to 100 | Qs to 100 | Qs to 100 | Qs to 100 | Qs to 100 | Qs to 100 | Qs to 100 | Qs to 100 | Qs to 100 |
| | Sodium Chloride | 0.40 | 0.40 | 0.40 | 0.40 | 0.40 | 0.40 | 0.40 | 0.40 | 0.40 |
| | UV WAT | | 5.0 | 5.0 | 5.0 | 5.0 | 5.0 | 5.0 | 0.0 | 5.0 |
| Part D | Cetyl PEG/PPG-10/1 Dimethicone | 2.0 | 2.0 | 2.0 | 2.0 | 2.0 | 2.0 | 2.0 | 2.0 | 2.0 |
| | Microcrystalline Wax | 0.90 | 0.90 | 0.90 | 0.90 | 0.90 | 0.90 | 0.90 | 0.90 | 0.90 |
| | Hydrogenated Castor Oil | 0.60 | 0.60 | 0.60 | 0.60 | 0.60 | 0.60 | 0.60 | 0.60 | 0.60 |
| | Decyl Oleate | 10.5 | 5.0 | 5.0 | 5.0 | 5.0 | 5.0 | 5.0 | 5.0 | 5.0 |
| | Caprylic/Capric Triglyceride | 10.0 | 0.0 | 0.0 | 0.0 | 0.0 | 0.0 | 0.0 | 0.0 | 0.0 |
| | Jojoba (Buxus Chinensis) Oil | 2.0 | 2.0 | 2.0 | 2.0 | 2.0 | 2.0 | 2.0 | 2.0 | 2.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | UV filter according to the present invention | | 3.0 | 15.0 | 5.0 | 10.0 | 5.0 | 5.0 | 5.0 | 15.0 |
| | Preservative, Parfum | q.s. | q.s. | q.s. | q.s. | q.s. | q.s. | q.s. | q.s. | q.s. |

| W/O/W Emulsion | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Glycerylstearate | 3.0 | 3.0 | 3.0 | 3.0 | 3.0 | 3.0 | 3.0 | 3.0 | 3.0 |
| | PEG-100-Stearate | 0.75 | 0.75 | 0.75 | 0.75 | 0.75 | 0.75 | 0.75 | 0.75 | 0.75 |
| | Behenylalcohol | 2.0 | 2.0 | 2.0 | 2.0 | 2.0 | 2.0 | 2.0 | 2.0 | 2.0 |
| | Caprylic-/Capric-Triglyceride | 8.0 | 0.0 | 0.0 | 0.0 | 0.0 | 0.0 | 0.0 | 0.0 | 0.0 |
| | Octyldodecanol | 5.0 | 0.0 | 0.0 | 0.0 | 0.0 | 0.0 | 0.0 | 0.0 | 0.0 |
| | C12-15 Alkylbenzoate | 3.0 | 0.0 | 0.0 | 0.0 | 0.0 | 0.0 | 0.0 | 0.0 | 0.0 |
| | UV filter according to the present invention | | 3.0 | 15.0 | 5.0 | 10.0 | 5.0 | 5.0 | 5.0 | 15.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | UV WAT | | 5.0 | 5.0 | 5.0 | 5.0 | 5.0 | 5.0 | 0.0 | 5.0 |
| | Dodecanedioic Acid | 1.0 | 1.0 | 1.0 | 1.0 | 1.0 | 1.0 | 1.0 | 1.0 | 1.0 |
| | Magnesium Sulfate | 0.80 | 0.80 | 0.80 | 0.80 | 0.80 | 0.80 | 0.80 | 0.80 | 0.80 |
| | EDTA | 0.10 | 0.10 | 0.10 | 0.10 | 0.10 | 0.10 | 0.10 | 0.10 | 0.10 |
| | Phenoxyethanol | q.s. | q.s. | q.s. | q.s. | q.s. | q.s. | q.s. | q.s. | q.s. |
| | Parfume | q.s. | q.s. | q.s. | q.s. | q.s. | q.s. | q.s. | q.s. | q.s. |
| | Water | ad 100 | ad 10.0 | ad 100 | ad 100 | ad 100 | ad 100 | ad 100 | ad 100 | ad 100 |
| | PH-value adjusted to 6.0 | | | | | | | | | |

| Cream-to-powder | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Isoeicosane | 10.0 | 5.0 | 5.0 | 5.0 | 5.0 | 5.0 | 5.0 | 5.0 | 5.0 |
| | Polyisobutene | 2.0 | 2.0 | 2.0 | 2.0 | 2.0 | 2.0 | 2.0 | 2.0 | 2.0 |
| | UV filter according to the present invention | | 3.0 | 15.0 | 5.0 | 10.0 | 5.0 | 5.0 | 5.0 | 15.0 |
| | Cetearyl Octanoate | 20.5 | 10.0 | 5.0 | 10.0 | 5.0 | 5.0 | 5.0 | 5.0 | 10.0 |
| | Oleyl alcohol | 5.0 | 5.0 | 5.0 | 5.0 | 5.0 | 5.0 | 5.0 | 5.0 | 5.0 |
| | Ceresin | 4.50 | 4.50 | 4.50 | 4.50 | 4.50 | 4.50 | 4.50 | 4.50 | 4.50 |
| | Talc | 11.60 | 11.60 | 11.60 | 11.60 | 11.60 | 11.60 | 11.60 | 11.60 | 11.60 |
| | Polyethylene | 4.0 | 4.0 | 4.0 | 4.0 | 4.0 | 4.0 | 4.0 | 4.0 | 4.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | Silica | 17.75 | 17.75 | 17.75 | 17.75 | 17.75 | 17.75 | 17.75 | 17.75 | 17.75 |
| | Calcium Aluminum Borosilicate (and) Bismuth oxychloride | 1.0 | 1.0 | 1.0 | 1.0 | 1.0 | 1.0 | 1.0 | 1.0 | 1.0 |
| | Iron Oxides | 3.40 | 3.40 | 3.40 | 3.40 | 3.40 | 3.40 | 3.40 | 3.40 | 3.40 |
| | Tocopherol Acetate | 0.25 | 0.25 | 0.25 | 0.25 | 0.25 | 0.25 | 0.25 | 0.25 | 0.25 |

| Foundations: Anhydrous forms | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Isononyl Isononanoate | qsp | qsp | qsp | qsp | qsp | qsp | qsp | qsp | qsp |
| | UV filter according to the present invention | 3.0 | 3.0 | 15.0 | 5.0 | 10.0 | 5.0 | 5.0 | 5.0 | 15.0 |
| | Sorbitan Sesquioleate | 0.50 | 0.50 | 0.50 | 0.50 | 0.50 | 0.50 | 0.50 | 0.50 | 0.50 |
| | Cyclopentasiloxane | 15.0 | 15.0 | 15.0 | 15.0 | 15.0 | 15.0 | 15.0 | 15.0 | 15.0 |
| | Cylopentasiloxane (and) Quaternium-18 Hectorite | 15.0 | 15.0 | 15.0 | 15.0 | 15.0 | 15.0 | 15.0 | 15.0 | 15.0 |
| | Talc | 5.0 | 5.0 | 5.0 | 5.0 | 5.0 | 5.0 | 5.0 | 5.0 | 5.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | Iron oxides | 2.50 | 2.50 | 2.50 | 2.50 | 2.50 | 2.50 | 2.50 | 2.50 | 2.50 |
| | PVP / eicosane copolymer | 1.0 | 1.0 | 1.0 | 1.0 | 1.0 | 1.0 | 1.0 | 1.0 | 1.0 |
| | Tocopherol Acetate | 0.50 | 0.50 | 0.50 | 0.50 | 0.50 | 0.50 | 0.50 | 0.50 | 0.50 |

| Pickering Emulsions | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| | Octyidodecanol | 5.50 | 0.0 | | | | | | | |
| | UV filter according to the present invention | 3.0 | 3.0 | 15.0 | 5.0 | 10.0 | 5.0 | 5.0 | 5.0 | 15.0 |
| | C12-15-Alkyl Benzoate | 6.50 | 0.0 | 0.0 | 0.0 | 0.0 | 0.0 | 0.0 | 0.0 | 0.0 |
| | Dicaprylyl Ether | 5.50 | 0.0 | 0.0 | 0.0 | 0.0 | 0.0 | 0.0 | 0.0 | 0.0 |
| | Hydroxyoctacosanyl Hydroxystearate | 2.0 | 2.0 | 2.0 | 2.0 | 2.0 | 2.0 | 2.0 | 2.0 | 2.0 |
| | Disteardimonium Hectorit | 1.0 | 1.0 | 1.0 | 1.0 | 1.0 | 1.0 | 1.0 | 1.0 | 1.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | UV WAT | | 5.0 | 5.0 | 5.0 | 5.0 | 5.0 | 5.0 | 0.0 | 5.0 |
| | Bariumsulfate | 0.50 | 0.50 | 0.50 | 0.50 | 0.50 | 0.50 | 0.50 | 0.50 | 0.50 |
| | Boron Nitride | 2.0 | 2.0 | 2.0 | 2.0 | 2.0 | 2.0 | 2.0 | 2.0 | 2.0 |
| | NaCl | 1.0 | 1.0 | 1.0 | 1.0 | 1.0 | 1.0 | 1.0 | 1.0 | 1.0 |
| | Glycerine | 10.0 | 10.0 | 10.0 | 10.0 | 10.0 | 10.0 | 10.0 | 10.0 | 10.0 |
| | Trisodlum EDTA | 1.0 | 1.0 | 1.0 | 1.0 | 1.0 | 1.0 | 1.0 | 1.0 | 1.0 |
| | Porpylene Carbonate | 0.33 | 0.33 | 0.33 | 0.33 | 0.33 | 0.33 | 0.33 | 0.33 | 0.33 |
| | Methylparabene | 0.20 | 0.20 | 0.20 | 0.20 | 0.20 | 0.20 | 0.20 | 0.20 | 0.20 |
| | Phenoxyethanol | 0.50 | 0.50 | 0.50 | 0.50 | 0.50 | 0.50 | 0.50 | 0.50 | 0.50 |
| | Hexamidine Diisethionate | 0.08 | 0.08 | 0.08 | 0.08 | 0.08 | 0.08 | 0.08 | 0.08 | 0.08 |
| | Parfume | 0.40 | 0.40 | 0.40 | 0.40 | 0.40 | 0.40 | 0.40 | 0.40 | 0.40 |
| | Water | ad 100 | ad 100 | ad 100 | ad 100 | ad 100 | ad 100 | ad 100 | ad 100 | ad 100 |

| Microemulsion Lotion | | A | B | C | D | E | E | G | H | I |
|---|---|---|---|---|---|---|---|---|---|---|
| | INCI-Name | % w/w | % w/w | % w/w | % w/w | % w/w | % w/w | % w/w | % w/w | % w/w |
| | PPG-26-Buteth-26 | 3.0 | 3.0 | 3.0 | 3.0 | 3.0 | 3.0 | 3.0 | 3.0 | 3.0 |
| | Ceteareth 20 | 10.0 | 10.0 | 10.0 | 10.0 | 10.0 | 10.0 | 10.0 | 10.0 | 10.0 |
| | C12-15 Alkyl Benzoate | 5.0 | 0.0 | 0.0 | 0.0 | 0.0 | 0.0 | 0.0 | 0.0 | 0.0 |
| | UV filter according to the present invention | 3.0 | 3.0 | 15.0 | 5.0 | 10.0 | 5.0 | 5.0 | 5.0 | 15.0 |
| | Oleth-5 | 4.0 | 4.0 | 4.0 | 4.0 | 4.0 | 4.0 | 4.0 | 4.0 | 4.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| | PPG-11 Stearyl Ether | 10.0 | 0.0 | 0.0 | 0.0 | 0.0 | 0.0 | 0.0 | 0.0 | 0.0 |
| | UV WAT | | 5.0 | 5.0 | 5.0 | 5.0 | 5.0 | 5.0 | 0.0 | 5.0 |
| | Aluminum Chlorohydrex PG | 15.0 | 15.0 | 15.0 | 15.0 | 15.0 | 15.0 | 15.0 | 15.0 | 15.0 |
| | Water | Qs to 100 | Qs to 100 | Qs to 100 | Qs to 100 | Qs to 100 | Qs to 100 | Qs to 100 | Qs to 100 | Qs to 100 |

| Cationic O/W sun cream | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Palmitamidopropyltrimoniu m Chloride | 6.0 | 6.0 | 6.0 | 6.0 | 6.0 | 6.0 | 6.0 | 6.0 | 6.0 |
| | Stearyl Alcohol | 2.0 | 2.0 | 2.0 | 2.0 | 2.0 | 2.0 | 2.0 | 2.0 | 2.0 |
| | Isocetyl Palmitate | 4.0 | 0.0 | 0.0 | 0.0 | 0.0 | 0.0 | 0.0 | 0.0 | 0.0 |
| | Decyl Cocoate | 3.0 | 0.0 | 0.0 | 0.0 | 0.0 | 0.0 | 0.0 | 0.0 | 0.0 |
| | C12-15 Alkyl Benzoate | 3.0 | 0.0 | 0.0 | 0.0 | 0.0 | 0.0 | 0.0 | 0.0 | 0.0 |
| | UV filter according to the present invention | 3.0 | 3.0 | 15.0 | 5.0 | 10.0 | 5.0 | 5.0 | 5.0 | 15.0 |
| | Cetyl Dimethicone | 0.75 | 0.75 | 0.75 | 0.75 | 0.75 | 0.75 | 0.75 | 0.75 | 0.75 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| Part B | Aqua | Qs to 100 | Qs to 100 | Qs to 100 | Qs to 100 | Qs to 100 | Qs to 100 | Qs to 100 | Qs to 100 | Qs to 100 |
| | Glycerin | 3.0 | 3.0 | 3.0 | 3.0 | 3.0 | 3.0 | 3.0 | 3.0 | 3.0 |
| | Trisodium EDTA | 0.10 | 0.10 | 0.10 | 0.10 | 0.10 | 0.10 | 0.10 | 0.10 | 0.10 |
| | UV WAT | | 5.0 | 5.0 | 5.0 | 5.0 | 5.0 | 5.0 | 0.0 | 5.0 |
| Part C | Capryl/Capramidopropyl Betaine | 8.0 | 8.0 | 8.0 | 8.0 | 8.0 | 8.0 | 8.0 | 8.0 | 8.0 |
| | Phenoxyethanol (and) Methylparaben (and) Ethylparaben (and) Butylparaben (and) Propylparaben (and) Isobutylparaben | 0.70 | 0.7 0 | 0.70 | 0.70 | 0.70 | 0.70 | 0.70 | 0.70 | 0.70 |
| | Tocopheryl Acetate | 0.80 | 0.8 0 | 0.80 | 0.80 | 0.80 | 0.80 | 0.80 | 0.80 | 0.80 |

| Si/W sun cream | | A | B | C | D | E | F | G | H | I |
|---|---|---|---|---|---|---|---|---|---|---|
| | INCI-Name | % w/w | % w/w | % w/w | % w/w | % w/w | % w/w | % w/w | % w/w | % w/w |
| Part A | Cyclopentasiloxane (and) Dimethicone/Vinyl Dimethicone Crosspolymer | 2.0 | 2.0 | 2.0 | 2.0 | 2.0 | 2.0 | 2.0 | 2.0 | 2.0 |
| | Dimethicone (and) Dimethicone/Vinyl Dimethicone Crosspolymer | 3.0 | 3.0 | 3.0 | 3.0 | 3.0 | 3.0 | 3.0 | 3.0 | 3.0 |
| | Cyclopentasiloxane | 10.0 | 10.0 | 10.0 | 10.0 | 10.0 | 10.0 | 10.0 | 10.0 | 10.0 |
| | UV filter according to the present invention | 3.0 | 3.0 | 15.0 | 5.0 | 10.0 | 5.0 | 5.0 | 5.0 | 15.0 |
| | UV SOL | | 5.0 | 10.0 | 5.0 | 10.0 | 5.0 | 10.0 | 5.0 | 5.0 |
| | UV LIQ | | 5.0 | 5.0 | 10.0 | 10.0 | 15.0 | 15.0 | 15.0 | 0.0 |
| Part B | 1.3-Butylen Glycol | 3.0 | 3.0 | 3.0 | 3.0 | 3.0 | 3.0 | 3.0 | 3.0 | 3.0 |
| | Polyglyceryl-3 Disiloxane Dimethicone | 0.60 | 0.60 | 0.60 | 0.60 | 0.60 | 0.60 | 0.60 | 0.60 | 0.60 |
| | Polyglyceryl-3 Polydimethylsiloxyethyl Dimethicone | 0.30 | 0.30 | 0.30 | 0.30 | 0.30 | 0.30 | 0.30 | 0.30 | 0.30 |
| | Acrylamide/Sodium Acryloyldimethyltaurate Copolymer (and) Isohexadecane (and) Polysorbate 80 | 0.60 | 0.60 | 0.60 | 0.60 | 0.60 | 0.60 | 0.60 | 0.60 | 0.60 |
| | Ammonium Acryloyldimethyltaurate/VP Copolymer | 0.65 | 0.65 | 0.65 | 0.65 | 0.65 | 0.65 | 0.65 | 0.65 | 0.65 |
| | Sodium Chloride | 0.10 | 0.10 | 0.10 | 0.10 | 0.10 | 0.10 | 0.10 | 0.10 | 0.10 |
| | Aqua | Qs to 100 | Qs to 100 | Qs to 100 | Qs to 100 | Qs to 100 | Qs to 100 | Qs to 100 | Qs to 100 | Qs to 100 |
| | UV WAT | | 5.0 | 5.0 | 5.0 | 5.0 | 5.0 | 5.0 | 0.0 | 5.0 |

The invention relates also to cosmetic compositions that comprise the UV absorbers according to the invention. The cosmetic compositions are suitable especially as UV filters, that is to say for the protection of organic materials that are sensitive to ultraviolet light, especially skin and hair, against the damaging action of UV radiation.

The cosmetic compositions contain, for example, from 0.1 to 30 % by weight, preferably from 0.1 to 15 % by weight and especially from 0.5 to 10 % by weight, based on the total weight of the composition, of one or more UV absorbers and at least one cosmetically tolerable adjuvant.

The cosmetic compositions can be prepared by physically mixing the UV absorber(s) with the adjuvant using customary methods, for example by simply stirring together the individual components, especially by making use of the dissolution properties of already known cosmetic UV absorbers, for example OMC, salicylic acid isooctyl ester, *inter alia.* The UV absorber can be used, for example, without further treatment.

The cosmetic compositions may be, for example, creams, gels, lotions, alcoholic and aqueous/alcoholic solutions, emulsions, wax/fat compositions, stick preparations, powders or ointments.

The compositions according to the invention, for example creams, gels, lotions, alcoholic and aqueous/alcoholic solutions, emulsions, wax/fat compositions, stick preparations, powders or ointments, may in addition contain, as further adjuvants and additives, mild surfactants, super-fatting agents, pearlescent waxes, consistency regulators, thickeners, polymers, silicone compounds, fats, waxes, stabilisers, biogenic active ingredients, deodorising active ingredients, anti-dandruff agents, film formers, swelling agents, further UV light-protective factors, antioxidants, hydrotropic agents, preservatives, insect repellents, self-tanning agents, solubilisers, perfume oils, colourants, bacteria-inhibiting agents and the like.

Cosmetic formulations according to the invention are contained in a wide variety of cosmetic preparations. There come into consideration, for example, especially the following preparations: skin-care preparations, bath preparations, skin-care preparations, cosmetic personal care preparations, foot-care preparations, light-protective preparations, skin-tanning preparations, depigmenting preparations, insect-repellents, deodorants, antiperspirants, preparations for cleansing and caring for blemished skin, hair-removal preparations in chemical form (depilation), shaving preparations, fragrance preparations, cosmetic hair-treatment preparations.

The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic compositions for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection oils, sun protection milk and sun protection preparations in the form of a spray.

Of special importance as cosmetic compositions for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

The cosmetic preparation according to the invention contains from 0.1 to 15 % by weight, preferably from 0.5 to 10 % by weight, based on the total weight of the composition, of a UV absorber of formula (1) or of a mixture of UV absorbers and a cosmetically tolerable adjuvant.

The cosmetic preparation can be prepared by physically mixing the UV absorber or UV absorbers with the adjuvant using conventional methods, for example by simply stirring the individual components together.

The cosmetic preparation according to the invention can be formulated as a water-in-oil or oil-in-water emulsion, as an oil-in-alcohol lotion, as a vesicular dispersion of an ionic or nonionic amphiphilic lipid, as a gel, solid stick or as an aerosol formulation.

As a water-in-oil or oil-in-water emulsion, the cosmetically tolerable adjuvant preferably contains from 5 to 50 % of an oil phase, from 5 to 20 % of an emulsifier and from 30 to 90 % water. The oil phase can comprise any oil suitable for cosmetic formulations, for example one or more hydrocarbon oils, a wax, a natural oil, a silicone oil, a fatty acid ester or a fatty alcohol. Preferred mono- or poly-ols are ethanol, isopropanol, propylene glycol, hexylene glycol, glycerol and sorbitol.

For the cosmetic preparation according to the invention it is possible to use any conventionally usable emulsifier, for example one or more ethoxylated esters of natural derivatives, for example polyethoxylated esters of hydrogenated castor oil, or a silicone oil emulsifier, for example silicone polyol; an unethoxylated or ethoxylated fatty acid soap; an ethoxylated fatty alcohol; an unethoxylated or ethoxylated sorbitan ester; an ethoxylated fatty acid; or an ethoxylated glyceride.

The cosmetic preparation according to the invention is distinguished by excellent protection of human skin against the damaging effect of sunlight.

In the following Examples percentages relate to weight. The amounts of the benzylidene malonates compounds used relate to the pure substance.

### Preparation Examples

### Example 1: 4-Methoxybenzylidene-di-(2-methylbutyl)-malonate

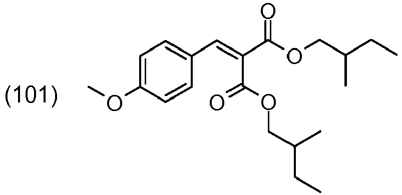

In a flask with a distillation head a mixture of diethyl malonate (103 g, 0.637 mol), 2-methyl-1-butanol (284 g, 3.18 mol) and conc. sulfuric acid (95%, 12.9 g, 0.125 mol) is heated by an oil bath (bath temperature 160°C) for 3 h. During this time 120 ml of distillate are collected. The reaction mixture is cooled to room temperature and washed with water. After removing the excess of 2-methl-1-butanol under reduced pressure using a rotary evaporator di-(2-methylbutyl)-malonate (153 g, 98%) is obtained as a colourless liquid. The compound may be further purified by vacuum distillation (bp. 115-125°C/5 mbar).
¹H-NMR (360 MHz, CDCl₃): δ = 0.86-0.93 (m, 12 H), 1.12-1.24 (m, 2 H), 1.36-1.48 (m, 2 H), 1.65-1.78 (m, 2 H), 3.37 (s, 2 H), 3.93 (dd, *J* = 6.5 Hz, 10.5 Hz, 2 H), 4.03 (dd, *J* = 6.5 Hz, 10.5 Hz, 2 H); ¹³C-NMR (90 MHz, CDCl₃): δ = 11.06, 16.19, 25.84, 33.98, 41.62, 70.00, 166.6 ppm.

In a flask with a Dean-Stark apparatus a mixture of 4-methoxybenzaldehyde (67.4 g, 0.495 mol), di-(2-methylbutyl)-malonate (110 g, 0.450 mol) and n-hexylamine (4.7 g, 0.045 mol) is heated to reflux for 4 h. The reaction mixture is cooled to room temperature and the toluene is removed under reduced pressure using a rotary evaporator. Purification by Kugelrohr distillation under high vacuum yields 4-methoxybenzylidene-di-(2-methylbutyl)-malonate (136 g, 83%).
¹H-NMR (360 MHz, CDCl₃): δ = 0.87-0.95 (m, 12 H), 1.11-1.28 (m, 2 H), 1.36-1.51 (m, 2 H), 1.65-1.85 (m, 2 H), 3.83 (s, 3 H), 4.01-4.17 (m, 4 H), 6.88 (d, *J* = 8.5 Hz, 2 H), 7.41 (d, *J* = 8.5 Hz, 2 H), 7.66 (s, 1 H) ppm; ¹³C-NMR (90 MHz, CDCl₃): δ = 11.06, 11.14, 16.29, 25.85, 25.92, 33.90, 34.14, 55.30, 69.86, 70.27, 114.3, 123.7, 125.4, 131.5, 141.7, 161.5, 164.5, 167.4 ppm.

## Claims

1. A composition for use in the protection of human skin against UV radiation comprising a benzylidene malonate of formula (1) 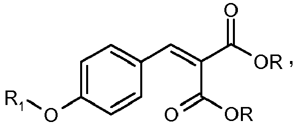 wherein
R₁ is methyl; and
R is

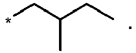

2. Cosmetic preparation comprising the compound of formula (1) according to claim 1 together with cosmetically tolerable carriers or adjuvants.

3. A preparation according to claim 2 that comprises further UV protective agents.

4. A preparation according to claim 3, wherein the UV protecting agents are selected from p-aminobenzoic acid derivatives, salicylic acid derivatives, benzophenone derivatives, dibenzoylmethane derivatives, diphenylacrylates, 3-imidazol-4-yl acrylic acid and esters; benzofuran derivatives, polymeric UV absorbers, cinnamic acid derivatives, camphor derivatives, hydroxyphenyltriazine compounds, benzotriazole compounds, trianilino-s-triazine derivatives, 2-phenylbenzimidazole-5-sulfonic acid and salts thereof; menthyl o-aminobenzoate; merocyanine derivatives; encapsulated UV absorbers, 4,4-diphenyl-1,3-butadiene derivatives, tris-(aryl) triazines, TiO₂, ZnO and mica.

5. Use of the benzylidene malonate as defined in claim 1 in a cosmetic formulation for the stabilization of other UV filters.

## Patentansprüche

1. Zusammensetzung zur Verwendung beim Schutz von menschlicher Haut gegen UV-Strahlung, umfassend ein Benzylidenmalonat der Formel (1) 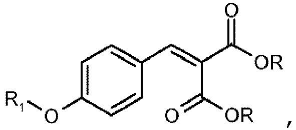 wobei
R₁ für Methyl steht und
R für 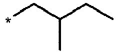 steht.

2. Kosmetische Zubereitung, umfassend die Verbindung der Formel (1) nach Anspruch 1 zusammen mit kosmetisch verträglichen Trägern oder Adjuvantien.

3. Zubereitung nach Anspruch 2, die ferner UV-Schutzmittel umfasst.

4. Zubereitung nach Anspruch 3, wobei die UV-Schutzmittel aus p-Aminobenzoesäurederivaten, Salicylsäurederivaten, Benzophenonderivaten, Dibenzoylmethanderivaten, Diphenylacrylaten, 3-Imidazol-4-ylacrylsäure und Estern davon; Benzofuranderivaten, polymeren UV-Absorbern, Zimtsäurederivaten, Campherderivaten, Hydroxyphenyltriazinverbindungen, Benzotriazolverbindungen, Trianilino-s-triazinderivaten, 2-Phenylbenzimidazol-5-sulfonsäure und Salzen davon; o-Aminobenzoesäurementhylester; Merocyaninderivaten; verkapselten UV-Absorbern, 4,4-Diphenyl-1,3-butadienderivaten, Tris(aryl)triazinen, TiO₂, ZnO und Glimmer ausgewählt sind.

5. Verwendung des Benzylidenmalonats gemäß Anspruch 1 in einer kosmetischen Formulierung zur Stabilisierung anderer UV-Filter.

## Revendications

1. Composition pour une utilisation dans la protection de la peau humaine contre le rayonnement UV comprenant un malonate de benzylidène de formule (1) 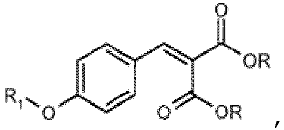
R₁ étant méthyle ; et
R étant

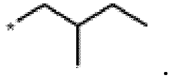

2. Préparation cosmétique comprenant le composé de formule (1) selon la revendication 1 conjointement avec des supports ou des adjuvants tolérables sur le plan cosmétique.

3. Préparation selon la revendication 2 qui comprend d'autres agents protecteurs contre les UV.

4. Préparation selon la revendication 3, les agents de protection contre les UV étant choisis parmi les dérivés de l'acide p-aminobenzoïque, les dérivés de l'acide salicylique, les dérivés de la benzophénone, les dérivés du dibenzoylméthane, les diphénylacrylates, l'acide 3-imidazol-4-ylacrylique et les esters de l'acide 3-imidazol-4-ylacrylique, les dérivés du benzofuranne, les absorbants des UV polymériques, les dérivés de l'acide cinnamique, les dérivés du camphre, les composés de type hydroxyphényltriazine, les composés de type benzotriazole, les dérivés de la trianilino-s-triazine, l'acide 2-phénylbenzimidazole-5-sulfonique et les sels correspondants ; l'o-aminobenzoate de menthyle ; les dérivés de mérocyanine ; les absorbants des UV encapsulés, les dérivés du 4,4-diphényl-1,3-butadiène, les tris(aryl)triazines, le TiO₂, le ZnO et le mica.

5. Utilisation du malonate de benzylidène tel que défini selon la revendication 1 dans une formulation cosmétique pour la stabilisation d'autres filtres UV.
